# EUROPÄISCHE PATENTSCHRIFT

(11) **EP 2 114 365 B1**
(45) Veröffentlichungstag und Bekanntmachung des Hinweises auf die Patenterteilung: **01.09.2010**
(21) Anmeldenummer: 08708532.0
(22) Anmeldetag: 31.01.2008
(51) Int. Cl.: A61K 8/81, A61Q 5/12

(54) **KATIONISCHES KONDITIONIERUNGSMITTEL**
CATIONIC CONDITIONING AGENT
PRODUIT DE CONDITIONNEMENT CATIONIQUE

(30) Priorität: 31.01.2007 EP 07101486
(43) Veröffentlichungstag der Anmeldung: 11.11.2009
(73) Patentinhaber: BASF SE, 67056 Ludwigshafen (DE)
(72) Erfinder: GARCIA CASTRO, Ivette, 67067 Ludwigshafen (DE); BELL, Hubertus, Peter, 68165 Mannheim (DE); PINNEKER, Olga, 67258 Hessheim (DE); WOOD, Claudia, 69469 Weinheim (DE); WINTER, Gabi, Shanghai 201204 (CN); MEIER, Nicole, 68307 Mannheim (DE)
(86) Internationale Anmeldenummer: PCT/EP2008/051221
(87) Internationale Veröffentlichungsnummer: WO 2008/092933

(56) Entgegenhaltungen:
- EP-A- 0 246 580
- EP-A- 0 911 018
- EP-A- 1 378 227
- WO-A-94/06403
- WO-A-94/06409
- WO-A-02/083073
- WO-A-2005/123014
- US-A- 6 110 451

## Beschreibung

Die vorliegende Erfindung betrifft Zusammensetzungen enthaltend wenigstens ein Polymer a) mit kationischen und/oder kationogenen Gruppen und einem Molekulargewicht M_{w} im Bereich von 10 000 bis 5 Millionen und wenigstens ein Polymer b) das in Gegenwart von Polymer a) hergestellt wurde.

Weiterhin betrifft die vorliegende Erfindung haut- und haarkosmetische Zusammensetzungen, insbesondere Shampoos und andere Haarpflegemittel enthaltend die Polymere a) und b), wobei b) in Gegenwart von a) hergestellt wurde. Die Erfindung betrifft demnach Zusammensetzungen zur Reinigung und/oder Pflege der Haare. Insbesondere betrifft die Erfindung außer Shampoos auch weitere Haarpflegemittel die ausgewählt sind aus der Gruppe bestehend aus Vorbehandlungsmitteln, Haarspülungen, Haarconditionern, Haarbalsamen, leave-on-Haarkuren, rinse-off-Haarkuren, Haarwässern, Pomaden, Frisiercremes, Frisierlotionen, Frisiergelen, Spitzenfluids, Hot-Oil-Treatments und Schaumkuren.

### Stand der Technik

Haarpflegemittel dienen in erster Linie dazu, die Trocken- und Nasskämmbarkeit, das Anfassgefühl, den Glanz und die Erscheinungsform des Haares zu verbessern sowie dem Haar antistatische Eigenschaften zu verleihen.

Ein Shampoo soll schäumen und das Haar gut reinigen, mild und verträglich sowie praktisch und angenehm in der Handhabung sein, es muss auch zur Pflege des Haares oder zur Beseitigung von Haar- und Kopfhautproblemen beitragen. Diese zusätzlichen Wirkungen und der selbstverständlich gewordene Reinigungseffekt sind für ein modernes Shampoo charakteristisch.

Reinigungs- und Schaumvermögen, Hautverträglichkeit, Verdickbarkeit und Hydrolysestabilität der einzelnen Inhaltsstoffe von Shampoos sind stark vom pH-Wert abhängig. Die in Shampoos verwendeten Inhaltsstoffe sollen diese Eigenschaften im neutralen und schwach sauren pH-Bereich (pH 5-7) optimal entfalten, allerdings auch außerhalb dieser pH-Wert-Bereiches keine deutlichen Leistungseinbußen aufzeigen. Die ausgewählten Inhaltsstoffe der Shampoos müssen chemisch stabil und mit allen anderen Rezepturbestandteilen verträglich sein, so dass z. B. keine Wirkungsminderungen oder Entmischungen stattfinden.

Von Shampoos wird eine ausreichende Reinigungskraft bei nicht zu starker Entfettungswirkung und gleichzeitig adäquater Milde erwartet. Wichtig ist, dass das Reinigungsvermögen und die weiteren gewünschten Tensideigenschaften sowohl in weichem als auch in hartem Wasser gegeben sind. Shampoos müssen gut haut- und schleimhautverträglich sein und dürfen daher unter den üblichen Anwendungsbedingungen keine aggressive Wirkung haben. Eine gute Reinigungsleistung muss nicht mit starker Schaumbildung verknüpft sein. Dennoch stellen Schaummenge und -qualität der Shampoos beim Waschen wichtige Kriterien für den Verbraucher dar, die von den Shampoos erfüllt werden müssen.

Shampoos haben neben der Reinigungswirkung auch konditionierende Wirkung, die durch den Gehalt von Konditioniermitteln im Shampoo gewährleistet wird. Konditioniermittel sind Hilfsstoffe, die auf das Haar aufziehen und auch nach dem Ausspülvorgang auf dem Haar verbleiben. Sie führen zu einer Verbesserung von Kämmbarkeit, Griff und Glanz des Haares. Bei bestimmten Haartypen (feinem Haar) bzw. Überdosierung können die Konditioniermittel jedoch auch zu einer unerwünschten Beschwerung des Haares führen. Formulierungstechnisch bedeutet dies, dass beim Einsatz von Konditioniermitteln stets auf eine ausgewogene Balance zwischen Konditionierleistung und Haarbeschwerung geachtet werden muss. Des Weiteren ist beim Einsatz von Konditioniermitteln auch darauf zu achten, dass die regelmäßige Anwendung des Produkts nicht zu einer stetig wachsenden Menge an Konditioniermitteln auf dem Haar führt (Build-up-Effekt). Schwierigkeiten bereitet oft die Bereitstellung von Produkten mit einem komplexen Eigenschaftsprofil. Solche komplexen Eigenschaftsprofile bedingen häufig den Einsatz zahlreicher Inhaltsstoffe in einem Präparat, was wiederum die Gefahr eine Unverträglichkeit gegenüber einem oder mehreren dieser Inhaltsstoffe beim Verbraucher impliziert.

Konditioniermittel in Shampoos sind vor allem Silikone und kationische Polymere. Silikone haben den Nachteil, dass sie meist wasserunlöslich sind und die Shampooformulierung durch Dispergiermittel stabilisiert werden muss. Diese Zusätze sind häufig unerwünscht. Ferner zeigen Silikone mitunter starke Build-up-Effekte und nach mehrmaliger Anwendung fühlt sich das Haar unangenehm beschwert an.

Viele kationische Polymere, die als Konditioniermittel in Shampoos Verwendung finden, wie beispielsweise kationische Cellulosederivate, bilden mit anionischen Tensiden der Shampooformulierung Tensid-Polymer-Komplexe, die bei hoher Ladungsdichte der Polymere wasserunlöslich sind. Deshalb werden üblicherweise kationische Polymere mit niedriger Ladungsdichte eingesetzt, damit sie in der Formulierung löslich sind.

Jedoch besitzen kationische Polymere mit hoher Ladungsdichte eine größere Affinität zum Haar, weshalb es wünschenswert ist, hochgeladene Polymere in Shampoos einzusetzen. Allerdings sind dann die Tensid-Polymer-Komplexe in der Formulierung unlöslich. Die Formulierung muss durch Zusatz von Dispergierhilfsmitteln stabilisiert werden.

WO 94/06403 beschreibt die Verwendung von u.a. Copolymeren aus N-Vinylpyrrolidon und 3-Methyl-1-vinylimidazoliumsalzen mit hoher Ladungsdichte in Kombination mit weiteren wasserunlöslichen Konditioniermitteln in Shampooformulierungen. Zur Stabilisierung der Formulierungen werden dementsprechend Dispergiermittel eingesetzt. WO 94/06409 und US 5580494 beschreiben Shampoozusammensetzungen auf Basis eines Alpha-Olefinsulfonates als Detergens und eines kationischen Polymers mit hoher Ladungsdichte, z.B. Copolymeren aus N-Vinylpyrrolidon und 3-Methyl-1-vinylimidazoliumsalzen als Konditioniermittel. Auch hier müssen zur Stabilisierung der Formulierungen Dispergierhilfsmittel zugesetzt werden.

EP-A 246 580 beschreibt, dass quaternisierte Vinylimidazol-Copolymerisate mit verschiedenen weiteren Monomeren als Haarkonditioniermittel verwendet werden. Die dort beschriebenen Polymere haben den Nachteil, dass sie im Falle eines geringen Anteils der quaternisierten Vinylimidazol-Monomeren in Gegenwart von Aniontensiden eine geringe Wirkung bzw. im Falle eines hohen Anteils des quaternisierten Vinylimidazols keine stabilen Dispersionen bilden.

EP-A 911 018 beschreibt die Verwendung von kationischen Copolymerisaten, erhältlich durch radikalisch initiierte Copolymerisation von
(a) 60 bis 99 Mol-%, vorzugsweise 65 bis 95 Mol-% und, besonders bevorzugt 70 bis 90 Mol-% eines gegebenenfalls substituierten 1-Vinylimidazols oder eines quaternisierten 1-Vinylimidazols,
(b) 1 bis 40 Mol-%, vorzugsweise 5 bis 35 Mol-%, besonders bevorzugt 10 bis 30 Mol-%, einer eine polymerisierbare Doppelbindung enthaltenden Säure oder deren Salzen und
(c) 0 bis 30 Mol-%, vorzugsweise 0 bis 20 Mol-%, besonders bevorzugt 0 bis 10 Mol-% eines weiteren radikalisch copolymerisierbaren Monomeren und anschließende Quaternisierung des Polymeren, sofern als Monomer (a) ein nicht quaternisiertes 1-Vinylimidazol eingesetzt wird, als Wirkstoffe in haarkosmetischen Zubereitungen, insbesondere als Konditioniermittel in Shampoos.

WO 02/083073 beschreibt wasserlösliche Polymerkomplexe, wobei bei der Polymerisation ein wasserlösliches Wirtspolymer vorgelegt und ein oder mehrere wasserlösliche Monomere polymerisiert werden, so dass sich ein stabiles einphasiges wässriges System bildet. Zusammensetzungen gemäß Anspruch 1 werden nicht beschrieben.

Die Zusammensetzungen, die aus dem Stand der Technik bekannt sind, weisen entweder zufriedenstellende haarpflegende Eigenschaften wie beispielsweise gute Nasskämmbarkeit oder zufriedenstellende formulierungstechnische Eigenschaften wie beispielsweise gute Stabilität auf.

Bei zahlreichen kationischen Polymeren aus dem Stand der Technik treten bei typischen ladungsausgleichenden stöchiometrischen Mengenverhältnissen von Polymer zu Tensid Unverträglichkeiten auf. Diese sind beispielsweise an nachlassender Transparenz, also an auftretender Trübung der Zusammensetzung erkennbar.

Aufgabe der vorliegenden Erfindung war die Bereitstellung von als Shampoo bzw. Hautreinigungsmittel vorliegenden wässrigen Zusammensetzungen, die sehr gute konditionierende Wirkung für Haare, Haut und Nägel aufweisen und gleichzeitig in Gegenwart von anionischen Tensiden langzeitstabil sind.

Es besteht daher weiterhin Bedarf an Shampoos und Haarpflegemitteln, die dem Haar gleichzeitig gute sensorisch erfassbare Eigenschaften, wie Elastizität; einen angenehmen Griff und Volumen verleihen, ohne dass eine gute konditionierende und reinigende Wirkung von einem unbefriedigenden fettigen und/oder klebrigen Erscheinungsbild der damit behandelten Haare begleitet wird und die als langzeitstabile Formulierungen mit weiterhin gutem Schaumbildungsvermögen und Schaumqualität vorliegen.

Es sollten Shampoos und Haarpflegemittel mit den vorgenannten Eigenschaften auf Basis möglichst weniger Einsatzstoffe entwickelt werden, da bei Mitteln aus dem Stand der Technik die Vielzahl an notwendigen Komponenten teilweise zu Hautirritationen, allergischen Reaktionen oder sonstigen Unverträglichkeiten führt. Insbesondere auch auf dem Gebiet der Kinder- und Baby-Shampoos und Haarpflegemitteln besteht ein Bedarf an Zusammensetzungen mit einer möglichst geringen Zahl an unterschiedlichen Inhaltsstoffen.

Eine Aufgabe der vorliegenden Erfindung war es, Zusammensetzungen bereitzustellen, die ohne zusätzliche Dispergierhilfsmittel langzeitstabile Shampooformulierungen mit Aniontensiden ergeben, die gleichzeitig ausgezeichnete Nasskämmbarkeiten auch ohne Verwendung von Silikonen zeigen.

Diese Aufgaben wurden gelöst durch Zusammensetzungen enthaltend
a) wenigstens ein Polymer a) mit kationischen und/oder kationogenen Gruppen und einem Molekulargewicht M_{w} im Bereich von 10 000 bis 5 Millionen und
b) wenigstens ein Polymer b) ausgewählt aus
   b1) Polymeren hergestellt in Gegenwart von Polymer a), die eine Verbindung der allgemeinen Formel I einpolymerisiert enthalten 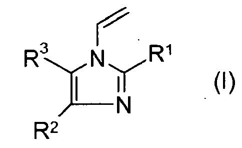 wobei R¹ bis R³ unabhängig voneinander Wasserstoff, C₁-C₄-Alkyl oder Phenyl bedeuten und
   b2) Kationischen Polymeren, hergestellt in Gegenwart von Polymer a) und wenigstens einem Salz und einpolymerisiert enthaltend wenigstens ein kationisches Monomer und wenigstens eine Verbindung der allgemeinen Formel I.

Polymer a) mit kationischen und/oder kationogenen Gruppen

Unter kationogenen Gruppen werden im Polymer kovalent gebundene Atomgruppen verstanden, die durch dem Fachmann bekannte chemische Umsetzungen in einen kationischen Ladungszustand, also in kationische Gruppen überführt werden können. Geeignete Polymere a) enthalten wenigstens eine olefinisch ungesättigte, radikalisch polymerisierbare Verbindung mit wenigstens einer kationischen und/oder kationogenen Gruppe pro Molekül einpolymerisiert.

Bevorzugt handelt es sich bei den kationogenen und/oder kationischen Gruppen um stickstoffhaltige Gruppen, wie primäre, sekundäre und tertiäre Aminogruppen sowie quaternäre Ammoniumgruppen. Vorzugsweise handelt es sich bei den stickstoffhaltigen Gruppen um tertiäre Aminogruppen oder quaternäre Ammoniumgruppen. Geladene kationische Gruppen lassen sich aus den Aminstickstoffen entweder durch Protonierung oder durch Quaternisierung mit Säuren oder Alkylierungsmitteln erzeugen. Dazu zählen z. B. Carbonsäuren, wie Milchsäure, oder Mineralsäuren, wie Phosphorsäure, Schwefelsäure und Salzsäure, oder als Alkylierungsmittel C₁-C₄-Alkylhalogenide oder -sulfate, wie Ethylchlorid, Ethylbromid, Methylchlorid, Methylbromid, Dimethylsulfat und Diethylsulfat.

Bevorzugt sind die olefinisch ungesättigten, radikalisch polymerisierbaren Verbindungen mit kationischer/kationogener Gruppe ausgewählt aus der Gruppe bestehend aus
- Estern α,β-ethylenisch ungesättigter Mono-und Dicarbonsäuren mit Aminoalkoholen, welche am Aminstickstoff mono-oder dialkyliert sein können,
- Amiden α,β-ethylenisch ungesättigter Mono-und Dicarbonsäuren mit Diaminen, welche mindestens eine primäre oder sekundäre Aminogruppe aufweisen,
- N,N-Diallylamin und Derivaten davon,
- vinyl-und allylsubstituierten Stickstoffheterocyclen,
- vinyl-und allylsubstituierten heteroaromatischen Verbindungen und
- Mischungen davon.

Ester α,β-ethylenisch ungesättigter Mono-und Dicarbonsäuren mit Aminoalkoholen, welche am Aminstickstoff mono-oder dialkyliert sein können:
Bevorzugte Aminoalkohole sind C₂-C₁₂-Aminoalkohole, welche am Aminstickstoff C₁-C₈-mono-oder -dialkyliert sind. Als Säurekomponente dieser Ester eignen sich z. B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden Acrylsäure, Methacrylsäure und deren Gemische eingesetzt. Besonders bevorzugt als Verbindungen c) sind N-Methylaminoethyl(meth)acrylat, N-Ethylaminoethyl(meth)acrylat, N-(n-Propyl)aminoethyl(meth)acrylat, N-(n-Butyl)aminoethyl(meth)acrylat, N-(tert.-Butyl)aminoethyl(meth)acrylat, N,N-Dimethylaminomethyl(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat und N,N-Dimethylaminocyclohexyl(meth)acrylat. Insbesondere werden N-(tert.-Butyl)aminoethylacrylat und N-(tert.-Butyl)aminoethylmethacrylat eingesetzt.

Amide α,β-ethylenisch ungesättigter Mono-und Dicarbonsäuren mit Diaminen, welche mindestens eine primäre oder sekundäre Aminogruppe aufweisen:
Bevorzugt sind Diamine, die eine tertiäre und eine primäre oder sekundäre Aminogruppe aufweisen. Bevorzugt werden als solche Monomere N-[2-(dimethylamino)ethyl]acrylamid, N-[2-(dimethylamino)ethyl]methacrylamid, N-[3-(dimethylamino)propyl]acrylamid, N-[3-(dimethylamino)propyl]methacrylamid, N-[4-(dimethylamino)butyl]acrylamid, N-[4-(dimethylamino)-butyl]methacrylamid, N-[2-(diethylamino)ethyl]acrylamid, N-[4-(dimethylamino)cyclohexyl]acrylamid und N-[4-(dimethylamino)cyclohexyl]methacrylamid eingesetzt, wobei N-[3-(dimethylamino)propyl]methacrylamid besonders bevorzugt ist.

Geeignete N,N-Diallylamine und Derivate davon sind beispielsweise N-Alkylderivate und die entsprechenden Säureadditionssalze; Alkyl steht dabei vorzugsweise für C₁-C₂₄-Alkyl. Bevorzugt sind z. B. N,N-Diallyl-N-methylamin und Diallyldimethylammoniumchlorid.

Vinyl-und allylsubstituierte Stickstoffheterocyclen:
Geeignete Beispiele hierfür sind N-Vinylimidazol, N-Vinylimidazol-Derivate, z. B. N-Vinyl-2-methylimidazol, vinyl-und allylsubstituierte heteroaromatische Verbindungen, wie 2-und 4-Vinylpyridin, 2-und 4-Allylpyridin, und die Salze davon.
Geeignet sind insbesondere N-Vinylimidazole der allgemeinen Formel (I), wie später unter Polymer b) definiert.

Zur Erzeugung einer kationischen Ladung von Polymer a) müssen die vorgenannten Monomere entweder in bereits quaternisiertem Zustand zur Polymerisation eingesetzt werden oder die Monomere nach Herstellung von Polymer a) im einpolymerisierten Zustand durch geeignete Umsetzungen wenigstens teilweise quaternisiert werden.

### Quaternisierung

Der Quaternisierungsgrad von Polymer a) (mol-% quaternisierte Gruppen von allen quaternisierbaren Gruppen) liegt bei wenigstens 60, bevorzugt bei wenigstens 70, besonders bevorzugt bei wenigstens 80 und insbesondere bei wenigstens 90%. Ganz besonders bevorzugt sind alle quaternisierbaren Gruppen quaternisiert, also ein Quaternisierungsgrad von 100 Mol-%, bezogen auf die quaternisierbaren Gruppen.

Zur Quaternisierung der Polymere a) und gegebenenfalls b) eignen sich beispielsweise Alkylhalogenide mit 1 bis 24 C-Atomen in der Alkylgruppe, z.B. Methylchlorid, Methylbromid, Methyliodid, Ethylchlorid, Ethylbromid, Propylchlorid, Hexylchlorid, Dodecylchlorid, Laurylchlorid, Propylbromid, Hexylbromid, Dodecylbromid, Laurylbromid und Benzylhalogenide, insbesondere Benzylchlorid und Benzylbromid. Zur Quaternierung mit langkettigen Alkylresten sind die entsprechenden Alkylbromide wie Hexylbromid, Dodecylbromid oder Laurylbromid bevorzugt. Weitere geeignete Quaternisierungsmittel sind Dialkylsulfate, insbesondere Dimethylsulfat oder Diethylsulfat.

Bevorzugte Quaternisierungsmittel sind Methylchlorid, Dimethylsulfat oder Diethylsulfat, wobei Methylchlorid und Dimethylsulfat besonders bevorzugt sind.

Die Quaternisierung der Monomere oder Polymere mit einem der genannten Quaternisierungsmittel erfolgt nach dem Fachmann bekannten, üblichen Methoden.

Beispiele für erfindungsgemäß geeignete Polymere a) sind explizit in WO 02/083073, Absätze [0054] bis [0056] beschrieben, worauf in vollem Umfang Bezug genommen wird.

Weitere erfindungsgemäß geeignete Polymere a) sind in US 6110451, Spalte 7, Z. 46 bis Spalte 9, Z.38 beschrieben, worauf ebenfalls in vollem Umfang Bezug genommen wird.

Erfindungsgemäß geeignet sind auch die von der CTFA (Cosmetic, Toiletry, and Fragrance Association, 1101 17th Street, NW Suite 300 Washington, D.C. 20036-4702) beschriebenen Polyquaternium-1 bis Polyquaternium-71.

Hierzu wird auch auf WO 02/083073, [0056] in vollem Umfang Bezug genommen. Bevorzugte Polymere a) enthalten Diallyldimethylammoniumchlorid (DADMAC) einpolymerisiert. Bevorzugt enthalten die Polymere a) zu wenigstens 30, besonders bevorzugt zu wenigstens 50, weiter bevorzugt zu wenigstens 70 und besonders bevorzugt zu wenigstens 90 Gew.-% DADMAC einpolymerisiert.

In einer bevorzugten Ausführungsform ist Polymer a) ein DADMAC-Homopolymer wie beispielsweise Polyquaternium-6. Polyquaternium-6 ist unter den Handelsnamen AEC^{®}Polyquaternium-6 (A & E Connock (Perfumery & Cosmetics) Ltd.), Agequat^{®}400 (CPS Chemical Company), Conditioner^{®}P6 (3V Group), Flocare^{®}C106 (SNF S.A.), Genamin^{®}PDAC (Clariant GmbH), Mackernium^{®}006 (Mclntyre Group Ltd), Merquat^{®}100 (Nalco Company), Merquat^{®}106 (Nalco Company), Mirapol^{®}100 (Rhodia Inc.), Octacare^{®}PQ6 (The Associated Octel Company Ltd), Rheocare^{®}CC6 (Cosmetic Rheologies, Ltd.), Rheocare^{®}CC6P (Cosmetic Rheologies, Ltd.), Ritaquta 6 (Rita Corporation), Salcare^{®}SC30 (Ciba Specialty Chemicals Corporation), Tinocare^{®}PQ-6H (Ciba Specialty Chemicals Corporation) oder Catiofast^{®}CS (BASF) erhältlich.

Polymer a) besitzt bevorzugt ein massenmittleres Molekulargewicht M_{w} im Bereich von 10 000-2 000 000, bevorzugt im Bereich von 50 000 bis 500 000, besonders bevorzugt im Bereich von 100 000 bis 200 000 g/mol.

### Polymer b1)

Polymer b1) wird hergestellt in Gegenwart von Polymer a) und enthält eine Verbindung der allgemeinen Formel I einpolymerisiert 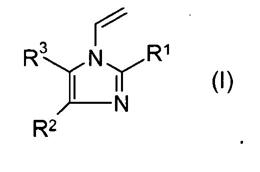 wobei R¹ bis R³ unabhängig voneinander Wasserstoff, C₁-C₄-Alkyl oder Phenyl bedeuten.

Bevorzugt liegt das massenmittlere Molekulargewicht M_{w} von Polymer b1) im Bereich von 1500 bis 500 000, besonders bevorzugt von 10 000 bis 100 000 und insbesondere von 30 000 bis 70 000 g/mol.

Die Menge an einpolymerisierter Verbindung der allgemeinen Formel I beträgt bevorzugt von 10 bis 100 Gew.-%, weiter bevorzugt von 20 bis 70 Gew.-%, besonders bevorzugt von 30 bis 60 Gew.-% und insbesondere 40 bis 60 Gew.-%

Beispiele für Verbindungen der allgemeinen Formel (I) sind folgender Tabelle zu entnehmen:

| R¹ | R² | R³ |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

| | | |
|---|---|---|
| Me = Methyl Ph = Phenyl | | |

Besonders bevorzugt ist N-Vinylimidazol (NVI), also die Verbindung der Formel I, wobei alle Reste R¹ bis R³ gleich Wasserstoff sind.

Insofern im Rahmen dieser Erfindung massenmittlere Molekulargewichte M_{w} angegeben sind, sind diese durch übliche, dem Fachmann bekannte Messmethoden bestimmt bzw. zu bestimmen. Bevorzugte Messmethoden zur Bestimmung von M_{w} sind Gelpermeationschromatographie (GPC) und Field Flow Fractionation (FFF). Der Fachmann weiss, welche Messbedingungen für welche Polymere anzuwenden sind.

In einer bevorzugten Ausführungsform liegt das einpolymerisierte NVI zu wenigstens 80 Mol-%, besonders bevorzugt zu wenigstens 90 Mol-%, ganz besonders bevorzugt zu wenigstens 95 Mol-% und insbesondere zu wenigstens 99 Mol-% in nicht-quaternisiertem Zustand vor. Am meisten bevorzugt liegt das einpolymerisierte NVI zu 100 Mol-% in nicht-quaternisiertem Zustand vor.

Polymer b1) enthält in einer bevorzugten Ausführungsform der Erfindung
i) im Bereich von 10 bis 100 Gew.-% wenigstens einer Verbindung der allgemeinen Formel I und
ii) im Bereich von 0 bis 90 Gew.-% wenigstens einer Verbindung der allgemeinen Formel II 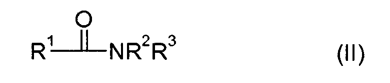
wobei R¹ für eine Gruppe der Formel CH₂=CR⁴- mit R⁴ = H oder C₁-C₄-Alkyl steht und R² und R³ unabhängig voneinander für H, Alkyl, Cycloalkyl, Heterocycloalkyl, Aryl oder Hetaryl stehen oder R² und R³ gemeinsam mit dem Stickstoffatom, an das sie gebunden sind, für einen fünf- bis achtgliedrigen Stickstoff-Heterocyclus stehen oder R² für eine Gruppe der Formel CH₂=CR⁴- steht und R¹ und R³ unabhängig voneinander für H, Alkyl, Cycloalkyl, Heterocycloalkyl, Aryl oder Hetaryl stehen oder R¹ und R³ gemeinsam mit der Amidgruppe, an die sie gebunden sind für ein Lactam mit 5 bis 8 Ringatomen stehen,
einpolymerisiert enthält.

Geeignete Verbindungen der Formel II sind ausgewählt unter primären Amiden α,β-ethylenisch ungesättigter Monocarbonsäuren, N-Vinylamiden gesättigter Monocarbonsäuren, N-Vinyllactamen, N-Alkyl-und N,N-Dialkylamiden α,β-ethylenisch ungesättigter Monocarbonsäuren und Mischungen davon.

Geeignete N-Alkyl-und N,N-Dialkylamide α,β-ethylenisch ungesättigter Monocarbonsäuren, die zusätzlich zu dem Carbonyl-Kohlenstoffatom der Amidgruppe höchstens 8 weitere Kohlenstoffatome aufweisen, sind beispielsweise N-Methyl(meth)acrylamid, N-Ethyl(meth)acrylamid, N-Propyl(meth)acrylamid, N-(n-Butyl)(meth)acrylamid, N-tert.-Butyl(meth)acrylamid, N,N-Dimethyl(meth)acrylamid, N,N-Diethyl(meth)acrylamid, Piperidinyl(meth)acrylamid, Morpholinyl(meth)acrylamid und Mischungen davon.

Besonders bevorzugt enthält Polymer b1) wenigstens ein N-Vinyllactam einpolymerisiert. Geeignete N-Vinyllactame sind beispielsweise unsubstituierte N-Vinyllactame und N-Vinyllactamderivate, die z.B. einen oder mehrere C₁-C₆-Alkylsubstituenten, wie Methyl, Ethyl, n-Propyl, Isopropyl, n-Butyl, sec.-Butyl, tert.-Butyl etc., aufweisen können. Dazu zählen z.B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam sowie deren Mischungen.

Insbesondere enthält Polymer b1) solche Verbindungen einpolymerisiert, bei denen in Formel II R² für CH₂=CH- und R¹ und R³, gemeinsam mit der Amidgruppe, an die sie gebunden sind, für ein Lactam mit 5 Ringatomen stehen.

Besonders bevorzugt enthält Polymer b1) solche Verbindungen einpolymerisiert, die ausgewählt sind unter Acrylsäureamid, Methacrylsäureamid, N-Vinylpyrrolidon, N-Vinylcaprolactam, N-Vinylformamid, N-Vinylacetamid und Mischungen davon. Am meisten bevorzugt ist N-Vinylpyrrolidon.

### Polymer b2)

Polymer b2) sind kationische Polymere, hergestellt in Gegenwart von Polymer a) und wenigstens einem Salz, die wenigstens ein kationisches Monomer und wenigstens eine Verbindung der vorstehenden allgemeinen Formel I einpolymerisiert enthalten. Geeignete kationische Monomere sind ausführlich vorstehend unter Polymer a) beschrieben.

Die Durchführung der Wasser-in-Wasser-Emulsionspolymerisation ist dem Fachmann beispielsweise aus den veröffentlichten Patentanmeldungen DE 10334262, DE 10338828, WO 2005/012378, WO 2006/018113, WO 98/54234 und DE 102005023799 bekannt.

Insbesondere sei auf die Ausführungen der WO 98/54234 verwiesen. Dort wird die Herstellung von wässrigen Polyvinylformamid-Dispersionen durch Polymerisation von Vinylformamid in Gegenwart eines wasserlöslichen polymeren Stabilisators wie beispielsweise PolyDADMAC und eines wasserlöslichen Salzes beschrieben. Zur Beschreibung der Herstellung von Polymer b2) in Gegenwart von Polymer a) sei auf die Offenbarung der WO 98/54234 bzw. EP 984990 hingewiesen, auf die in vollem Umfang Bezug genommen wird.

Beispiel 8 der vorliegenden Efindung beschreibt stellvertretend eine typische Vorgehensweise bei der Herstellung eines Polymers b2) durch eine Wasser-in-Wasser-Emulsionspolymerisation in Gegenwart von Polymer a) und eines Salzes.

Geeignete Salze sind ausführlich in den Schriften WO 98/14405 und WO 00/20470 beschrieben, auf die hiermit in vollem Umfang Bezug genommen wird.

Geeignete Salze sind demnach anorganische Salze wie Fluoride, Chloride, Sulfate, Phosphate oder Hydrogenphosphate von Metallionen oder Ammoniumionen. Typische Vertreter sind Natriumsulfat, Kaliumsulfat, Ammoniumsulfat, Magnesiumsulfat, Aluminiumsulfat, Natriumchlorid, Kalziumchlorid, Natriumdihydrogenphosphat, Diammomiumhydrogenphosphat, Dikaliumhydrogenphosphat, Kalziumphosphat, Natriumcitrat und Eisensulfat.

Chaotrope Salze, wie beispielsweise Thiocyanate, Perchlorate, Chlorate, Nitrate, Bromide und Iodide können ebenfalls verwendet werden. Typische Vertreter sind Kalziumnitrat, Natriumnitrat, Ammoniumnitrat, Aluminiumnitrat, Natriumthiocyanat und Natriumiodid.

Vorteilhaft werden Salze organischer C₁- bis C₁₅-Carbonsäuren, insbesondere die Alkalisalze, beispielsweise Natrium- oder Kaliumsalze oder Ammoniumsalze ein-, zwei- oder mehrbasiger organischer C₁- bis C₁₂-Carbonsäuren, wie beispielsweise Ameisensäure, Essigsäure, Zitronensäure, Oxalsäure, Malonsäure, Bernsteinsäure, Adipinsäure, Korksäure, Phthalsäure, Agaricinsäure, Trimesinsäure, 1,2,3-Propantricarbonsäure sowie 1,4-, 2,3- oder 2,6-Naphthalindicarbonsäure eingesetzt.

Besonders bevorzugt werden Citrate, insbesondere Natriumcitrat und Ammonium citrat eingesetzt.

Die vorgenannten Salze können einzeln oder als Mischungen von zwei oder mehr Salzen eingesetzt werden. Oftmals ist eine Mischung mehrerer Salze wirksamer als ein Salz allein, bezogen auf die eingesetzte Menge.

Die Salze werden in einer Menge zugesetzt, die 1 bis 100 Gew.-%, bevorzugt 10 bis 90 Gew.-% und besonders bevorzugt 15 bis 75 Gew.-% der Sättigungsmenge im wässrigen Reaktionsmedium unter Reaktionsbedingungen beträgt.

Unter 100 Gew.-% Sättigungsmenge im Reaktionsmedium ist diejenige Menge an Salz oder Salzen zu verstehen, die sich im wässrigen Reaktionsmedium der eingesetzten Monomere in Anwesenheit von Polymer a) sowie ggf. weiteren Hilfsstoffen bei der verwendeten Reaktionstemperatur gerade noch löst ohne zu präzipitieren.

Erfindungsgemäß kann die Gesamtmenge des wenigstens einen Salzes im Reaktionsmedium vorgelegt werden. Es ist aber auch möglich, gegebenenfalls lediglich eine Teilmenge des wenigstens einen Salzes im Reaktionsmedium vorzulegen und die gegebenenfalls verbliebene Restmenge bzw. die Gesamtmenge des wenigstens einen Salzes dem Reaktionsmedium unter Polymerisationsbedingungen zuzuführen. Dabei ist allerdings darauf zu achten, dass sowohl die zur Polymerisation eingesetzten Monomere (bis zu deren Abreaktion) wie auch das gebildete Polymer b2) unter Reaktionsbedingungen im wässrigen Reaktionsmedium stets als separate heterogene Phase vorliegen.

Von Bedeutung ist ferner, dass zur Herstellung von Polymer b2) Polymer a) optional auch in Kombination mit dem Fachmann geläufigen sogenannten neutralen Schutzkolloiden, wie beispielsweise Polyvinylalkohole, Poly-N-vinyl-2-pyrrolidon, Polyalkylenglykole, sowie Cellulose-, Stärke- oder Gelatinederivaten, eingesetzt werden kann. Dabei ist allerdings der Gewichtsanteil an optional eingesetzten neutralen Schutzkolloiden in der Regel geringer als der Gewichtsanteil an Polymer a) und beträgt oft < 5 Gew.-%, < 3 Gew.-% oder < 1 Gew.-%, jeweils bezogen auf die Gesamtmenge der zur Polymerisation für Polymer b2) eingesetzten Monomere.

Erfindungsgemäß kann die Gesamtmenge von Polymer a) ggf. in Kombination mit den neutralen Schutzkolloiden im Reaktionsmedium vorgelegt werden. Es ist aber auch möglich, gegebenenfalls lediglich eine Teilmenge von Polymer a) ggf. in Kombination mit den neutralen Schutzkolloiden im Reaktionsmedium vorzulegen und die gegebenenfalls verbliebene Restmenge bzw. die Gesamtmenge von Polymer a) ggf. in Kombination mit den neutralen Schutzkolloiden dem Reaktionsmedium unter Polymerisationsbedingungen zuzuführen.

### Weitere Monomere

Die Polymere a) und b) können weitere Monomere einpolymerisiert enthalten. Im Folgenden sind Beispiele für zur Copolymerisation geeignete Verbindungen angegeben: monoethylenisch ungesättigte Mono- und Dicarbonsäuren mit 3 bis 25 vorzugsweise 3 bis 6 C-Atomen, die auch in Form ihrer Salze oder Anhydride eingesetzt werden können wie beispielsweise Acrylsäure, Methacrylsäure, Ethacrylsäure, α-Chloracrylsäure, Crotonsäure, Maleinsäure, Maleinsäureanhydrid, Itaconsäure, Citraconsäure, Mesaconsäure, Glutaconsäure, Aconitsäure und Fumarsäure; Ester der (Meth)acryl-oder Ethacrylsäure, wie beispielsweise Methyl(meth)acrylat, Ethyl(meth)acrylat, n-und i-Propyl(meth)acrylat, Methylethacrylat, Ethylethacrylat, n-Propylethacrylat, i-Propylethacrylat, n-Butylethacrylat, tert.-Butylethacrylat, i-Butylethacrylat, n-Butyl(meth)acrylat, tert.-Butyt(meth)acrylat, i-Butyl(meth)acrylat, sec-Butyl(meth)acrylat, 2-Pentyl(meth)acrylat, 3-Pentyl(meth)acrylat, Isopentylacrylat, Neopentylacrylat, n-Octyl(meth)acrylat, 1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, O-leyl(meth)acrylat, Linolyl(meth)acrylat, Linolenyl(meth)acrylat, Stearyl(meth)acrylat, Lauryl(meth)acrylat, Phenoxyethylacrylat, 4-t-Butylcyclohexylacrylat, Cyclohexyl(meth)acrylat, Ureido(meth)acrylat, Tetrahydro-furfuryl(meth)acrylat und deren Mischungen;
Alkylvinylether der Formel H₂C=CH-O-R, wobei R C₁-C₃₀, bevorzugt C₈- bis C₂₂-Alkyl bedeutet, wie beispielsweise Methylvinylether, Ethylvinylether, n-Propylvinylether, i-Propylvinylether, n-Butylvinylether, sec-Butylvinylether, tert.-Butylvinylether, Dodecylvinylether, Octadecylvinylether etc..
Amide von Aminoalkoholen mit olefinisch ungesättigten Carbonsäuren wie beispielsweise 2-Hydroxypropylmethacrylamid, 3-Hydroxypropylacrylamid, 3-Hydroxypropylmethacrylamid, 3-Hydroxybutylacrylamid, 3-Hydroxybutylmethacrylamid, 4-Hydroxybutylacrylamid, 4-Hydroxybutylmethacrylamid, 6-Hydroxyhexylacrylamid, 6-Hydroxyhexylmethacrylamid, 3-Hydroxy-2-ethylhexylacrylamid und 3-Hydroxy-2-ethylhexylmethacrylamid;
Amide von Aminen mit olefinisch ungesättigten Carbonsäuren wie beispielsweise N-(n-Butyl)methacrylamid, N-(sek.-Butyl)methacrylamid, N-(tert.-Butyl)methacrylamid, N-(n-Pentyl)(meth)acrylamid, N-(n-Hexyl)(meth)acrylamid, N-(n-Heptyl)(meth)acrylamid, N-(n-Octyl)(meth)acrylamid, N-(tert.-Octyl)(meth)acrylamid N-(1,1,3,3-Tetramethylbutyl)(meth)acrylamid, N-Ethylhexyl(meth)acrylamid, N-(n-Nonyl)(meth)acrylamid, N-(n-Decyl)(meth)acrylamid, N-(n-Undecyl)(meth)acrylamid, N-Tridecyl(meth)acrylamid, N-Myristyl(meth)acrylamid, N-Pentadecyl(meth)acrylamid, N-Palmityl(meth)acrylamid, N-Heptadecyl(meth)acrylamid, N-Nonadecyl(meth)acrylamid, N-Arachinyl(meth)acrylamid, N-Behenyl(meth)acrylamid, N-Lignocerenyl(meth)acrylamid, N-Cerotinyl(meth)acrylamid, N-Melissinyl(meth)acrylamid, N-Palmitoleinyl(meth)acrylamid, N-Oleyl(meth)acrylamid, N-Linolyl(meth)acrylamid, N-Linolenyl(meth)acrylamid, N-Stearyl(meth)acrylamid, N-Lauryl(meth)acrylamid;
Vinylester wie beispielsweise Vinylacetat, Vinylpropionat, Vinylbutyrat und Mischungen davon;
Ethylen, Propylen, Isobutylen, Butadien, Styrol, α-Methylstyrol, Acrylnitril, Methacrylnitril, Vinylchlorid, Vinylidenchlorid, Vinylfluorid, Vinylidenfluorid und Mischungen davon.
Zur Copolymerisation geeignete Verbindungen, die C₈-C₃₀-, bevorzugt C₈-C₂₂- und besonders bevorzugt C₁₂-C₂₂-Alkylreste enthalten, sind beispielsweise Hexadecyl(meth)acrylat, Octadecyl(meth)acrylat, Hexadecylvinylether oder Octadecylvinylether.

Zur Copolymerisation geeignete Verbindungen die C₁-C₇-Alkylreste enthalten, sind beispielsweise Methyl(meth)acrylat, Ethyl(meth)acrylat, n-und i-Propyl(meth)acrylat, Methylethacrylat, Ethylethacrylat, n-Propylethacrylat, i-Propylethacrylat, n-Butylethacrylat, tert.-Butylethacrylat, i-Butylethacrylat, n-Butyl(meth)acrylat, tert.-Butyl(meth)acrylat, i-Butyl(meth)acrylat, sec-Butyl(meth)acrylat, 2-Pentyl(meth)acrylat, 3-Pentyl(meth)acrylat, Isopentylacrylat, Neopentylacrylat, C₁-C₇-Alkylvinylether e2) und deren Mischungen.

Die vorgenannten zur Copolymerisation geeigneten Verbindungen werden in einer Menge von 0, bevorzugt wenigstens 0,3, besonders bevorzugt wenigstens 0,5 und insbesondere wenigstens 1 und höchstens 30, bevorzugt höchstens 20, besonders bevorzugt höchstens 15 und insbesondere höchstens 12 Gew.-%, bezogen auf das Gesamtgewicht der zur Polymerisation eingesetzten Monomere, verwendet.

Am meisten bevorzugt werden diese Verbindungen in einer Menge von 1 bis 10 Gew.-%, bezogen auf das Gesamtgewicht der zur Polymerisation eingesetzten Monomere, zur Polymerisation verwendet.

Die Polymere a) und b) können auch wenigstens eine olefinisch ungesättigte, radikalisch polymerisierbare Verbindung mit wenigstens einer anionogenen und/oder anionischen Gruppe pro Molekül einpolymerisiert enthalten.

Solche Verbindungen können ausgewählt sein unter monoethylenisch ungesättigten Carbonsäuren, Sulfonsäuren, Phosphonsäuren und Mischungen davon.

Zu diesen Verbindungen zählen monoethylenisch ungesättigte Mono-und Dicarbonsäuren mit 3 bis 25 vorzugsweise 3 bis 6 C-Atomen, die auch in Form ihrer Salze oder Anhydride eingesetzt werden können. Beispiele hierfür sind Acrylsäure, Methacrylsäure, Ethacrylsäure, α-Chloracrylsäure, Crotonsäure, Maleinsäure, Maleinsäureanhydrid, Itaconsäure, Citraconsäure, Mesaconsäure, Glutaconsäure, Aconitsäure und Fumarsäure. Zu diesen Verbindungen zählen auch die Halbester von monoethylenisch ungesättigten Dicarbonsäuren mit 4 bis 10 vorzugsweise 4 bis 6 C-Atomen, z. B. von Maleinsäure wie Maleinsäuremonomethylester. Zu diesen Verbindungen zählen auch monoethylenisch ungesättigte Sulfonsäuren und Phosphonsäuren, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, 2-Acrylamido-2-methylpropansulfonsäure, Vinylphosphonsäure und Allylphosphonsäure. Zu diesen Verbindungen zählen auch die Salze der zuvor genannten Säuren, insbesondere die Natrium-, Kalium-und Ammoniumsalze sowie die Salze mit Aminen. Diese Verbindungen können als solche oder als Mischungen untereinander eingesetzt werden. Die angegebenen Gewichtsanteile beziehen sich sämtlich auf die Säureform. Wenn eine derartige anionische oder anionogene Verbindung eingesetzt werden soll, dann ist sie vorzugsweise ausgewählt aus der Gruppe bestehend aus Acrylsäure, Methacrylsäure, Ethacrylsäure, α-Chloracrylsäure, Crotonsäure, Maleinsäure, Maleinsäureanhydrid, Fumarsäure, Itaconsäure, Citraconsäure, Mesaconsäure, Glutaconsäure, Aconitsäure und Mischungen davon, besonders bevorzugt ausgewählt unter Acrylsäure, Methacrylsäure, Itaconsäure und Mischungen davon.

### Polymerisation

Zur Herstellung der Polymere b) in Gegenwart von Polymer a) kann das zu polymerisierende Gemisch sowohl mit Hilfe von Radikale bildenden Initiatoren als auch durch Einwirkung energiereicher Strahlung, worunter auch die Einwirkung energiereicher Elektronen verstanden werden soll, polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azo-bis-isobutyronitril, Azo-bis-(2-amidonopropan)dihydrochlorid oder 2-2'-Azo-bis-(2-methyl-butyronitril).

Geeignet sind auch Initiatormischungen oder Redox-Initiator-Systeme, wie z.B. Ascorbinsäure/Eisen(II)sulfat/Natriumperoxodisulfat, tert.-Butylhydroperoxid/Natriumdisulfit, tert.-Butylhydroperoxid/ Natriumhydroxymethansulfinat.

Bevorzugt werden organische Peroxide eingesetzt.

Die Polymerisation kann auch durch Einwirkung von ultravioletter Strahlung, gegebenenfalls in Gegenwart von UV-Initiatoren, durchgeführt werden. Für das Polymerisieren unter Einwirkung von UV-Strahlen setzt man die dafür üblicherweise in Betracht kommenden Photoinitiatoren bzw. Sensibilisatoren ein. Hierbei handelt es sich beispielsweise um Verbindungen wie Benzoin und Benzoinether, α-Methylbenzoin oder α-Phenylbenzoin. Auch sogenannte Triplett-Sensibilisatoren, wie Benzyldiketale, können verwendet werden. Als UV-Strahlungsquellen dienen beispielsweise neben energiereichen UV-Lampen, wie Kohlenbogenlampen, Quecksilberdampflampen oder Xenonlampen auch UV-arme Lichtquellen, wie Leuchtstoffröhren mit hohem Blauanteil.

Die verwendeten Mengen an Initiator bzw. Initiatorgemischen bezogen auf eingesetztes Monomer liegen zwischen 0,01 und 10 Gew.-%, vorzugsweise zwischen 0,1 und 8 Gew.-%, insbesondere zwischen 1 und 6 Gew.-%.

Die Herstellung von Polymer b1) kann beispielsweise als Lösungspolymerisation, Emulsionspolymerisation, umgekehrte Emulsionspolymerisation, Suspensionspolymerisation, umgekehrte Suspensionspolymerisation oder Fällungspolymerisation durchgeführt werden, ohne dass die verwendbaren Methoden darauf beschränkt sind.

Bevorzugt ist es, die Polymerisation als radikalische Lösungspolymerisation durchzuführen. Geeignete Lösungsmittel sind beispielsweise Wasser, Alkohole, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Glycerin, Dioxan, Butylacetat, Ethylacetat und Toluol, wobei Wasser, Alkohole und deren Mischungen besonders bevorzugt sind. Insbesondere wird als Lösungsmittel Wasser oder ein wässrig-ethanolisches Gemisch verwendet.

Der Feststoffgehalt, d.h. die gesamte Menge der vom Lösungsmittel verschiedenen in der Polymerisationslösung vorhandenen Substanzen, bezogen auf die gesamte Lösung, liegt üblicherweise im Bereich von 10 bis 40, bevorzugt im Bereich von 20 bis 30 Gew.-%.

Die Polymerisation erfolgt im Temperaturbereich von 30 bis 100°C, bevorzugt im Bereich von 50 bis 90°C. Sie wird üblicherweise unter atmosphärischem Druck durchgeführt, kann jedoch auch unter vermindertem oder erhöhtem Druck, vorzugsweise zwischen 1 und 5 bar, ablaufen.

Die Polymerisation kann auch halbkontinuierlich durchgeführt werden, indem man zunächst einen Teil, z.B. etwa 10 % des zu polymerisierenden Gemisches und Initiator vorlegt, das Gemisch auf Polymerisationstemperatur erhitzt und nach dem Anspringen der Polymerisation den Rest der zu polymerisierenden Mischung nach Fortschritt der Polymerisation zugibt.

In einer Ausführungsform der Erfindung kann die Polymerisation von Polymer b) in Gegenwart von Polymer a) auch derart erfolgen, dass die Polymerisation von Polymer b) in einem ersten Schritt bis zu einem Umsatz von 90, bevorzugt von 95, weiter bevorzugt von 99, besonders bevorzugt von 99,9 Gew.-%, bezogen auf die Gesamtmenge der Monomere, die zur Polymerisation von Polymer b) eingesetzt werden, in Abwesenheit von Polymer a) durchgeführt wird und in einem zweiten Schritt die nach dem ersten Schritt entstandene Reaktionsmischung enthaltend Polymer b) und die restlichen Monomere in Gegenwart von Polymer a) weiter polymerisiert wird.

Zu Beginn des zweiten Schrittes ist das Gewichtsverhältnis der Menge der restlichen Monomere aus der Herstellung von Polymer b) zur Menge von Polymer a) bevorzugt kleiner als 1:100, weiter bevorzugt kleiner als 1:150, besonders bevorzugt kleiner als 1:200.

### Regler

Die Herstellung der Polymere b) kann in Gegenwart mindestens eines Molekulargewichtsreglers erfolgen. Regler werden vorzugsweise in einer Einsatzmenge von 0,0005 bis 5 Gew.-%, besonders bevorzugt von 0,001 bis 2,5 Gew.-% und insbesondere von 0,01 bis 1,5 Gew.-% bezogen auf das Gesamtgewicht der Monomeren eingesetzt.

Als Regler werden allgemein Verbindungen mit hohen Übertragungskonstanten bezeichnet. Regler beschleunigen Kettenübertragungsreaktionen und bewirken damit eine Herabsetzung des Polymerisationsgrades der resultierenden Polymere, ohne die Bruttoreaktions-Geschwindigkeit zu beeinflussen.

Bei den Reglern kann man zwischen mono-, bi- oder polyfunktionalen Regler unterscheiden, je nach Anzahl der funktionellen Gruppen im Molekül, die zu einen oder mehreren Kettenübertragungsreaktionen führen können. Geeignete Regler werden beispielsweise ausführlich beschrieben von K.C. Berger und G. Brandrup in J. Brandrup, E.H. Immergut, Polymer Handbook, 3. Aufl., John Wiley & Sons, New York, 1989, S. II/81 - II/141.

Als Regler eignen sich beispielsweise Aldehyde wie Formaldehyd, Acetaldehyd, Propionaldehyd, n-Butyraldehyd oder Isobutyraldehyd.

Ferner können auch als Regler eingesetzt werden: Ameisensäure, ihre Salze oder Ester, wie Ammoniumformiat, 2,5-Diphenyl-1-hexen, Hydroxylammoniumsulfat, und Hydroxylammoniumphosphat.

Weitere geeignete Regler sind Halogenverbindungen, z. B. Alkylhalogenide, wie Tetrachlormethan, Chloroform, Bromtrichlormethan, Bromoform, Allylbromid, und Benzylverbindungen, wie Benzylchlorid oder Benzylbromid.

Weitere geeignete Regler sind Allylverbindungen, wie z. B. Allylalkohol, funktionalisierte Allylether, wie Allylethoxylate, Alkylallylether, oder Glycerinmonoallylether. Weiterhin können auch Alkohole wie beispielsweise Isopropanol als Regler eingesetzt werden.

Bevorzugt werden als Regler Verbindungen eingesetzt, die Schwefel in gebundener Form enthalten.

Verbindungen dieser Art sind beispielsweise anorganische Hydrogensulfite, Disulfite und Dithionite oder organische Sulfide, Disulfide, Polysulfide, Sulfoxide und Sulfone. Dazu zählen Di-n-butylsulfid, Di-n-octylsulfid, Diphenylsulfid, Thiodiglykol, Ethylthioethanol, Diisopropyldisulfid, Di-n-butyldisulfid, Di-n-hexyldisulfid, Diacetyldisulfid, Diethanolsulfid, Di-t-butyltrisulfid, Dimethylsulfoxid, Dialkylsulfid, Dialkyldisulfid und/oder Diarylsulfid.

Besonders bevorzugt sind organische Verbindungen, die Schwefel in gebundener Form enthalten.

Bevorzugt als Polymerisationsregler eingesetzte Verbindungen sind Thiole (Verbindungen, die Schwefel in Form von SH-Gruppen erhalten, auch als Mercaptane bezeichnet). Bevorzugt sind als Regler mono-, bi- und polyfunktionale Mercaptane, Mercaptoalkohole und/oder Mercaptocarbonsäuren.

Beispiele für diese Verbindungen sind Allylthioglykolate, Ethylthioglykolat, Cystein, 2-Mercaptoethanol, 1,3-Mercaptopropanol, 3-Mercaptopropan-1,2-diol, 1,4-Mercaptobutanol, Mercaptoessigsäure, 3-Mercaptopropionsäure, Mercaptobernsteinsäure, Thioglycerin, Thioessigsäure, Thioharnstoff und Alkylmercaptane wie n-Butylmercaptan, n-Hexylmercaptan oder n-Dodecylmercaptan.

Besonders bevorzugte Thiole sind Cystein, 2-Mercaptoethanol, 1,3-Mercaptopropanol, 3-Mercaptopropan-1,2-diol, Thioglycerin, Thioharnstoff.

Beispiele für bifunktionale Regler, die zwei Schwefelatome in gebundener Form enthalten, sind bifunktionale Thiole wie z. B. Dimercaptopropansulfonsäure (Natrium-Salz), Dimercaptobernsteinsäure, Dimercapto-1-propanol, Dimercaptoethan, Dimercaptopropan, Dimercaptobutan, Dimercaptopentan, Dimercaptohexan, Ethylenglykol-bisthioglykolate und Butandiol-bis-thioglykolat.

Beispiele für polyfunktionale Regler sind Verbindungen, die mehr als zwei Schwefelatome in gebundener Form enthalten. Beispiele hierfür sind trifunktionale und/oder tetrafunktionale Mercaptane.

Bevorzugte trifunktionale Regler sind trifunktionale Mercaptane, wie z. B. Trimethylolpropan-tris(2-mercaptoethanat, Trimethylolpropan-tris(3-mercaptopropionat), Trimethylolpropan-tris(4-mercaptobutanat), Trimethylolpropan-tris(5-mercaptopentanat), Trimethylolpropan-tris(6-mercaptohexanat), Trimethylolpropan-tris(2-mercaptoacetat), Glycerylthioglycolat, Glycerylthiopropionat, Glycerylthioethylat, Glycerylthiobutanat, 1,1,1-Propanetriyl-tris-(mercaptoacetat), 1,1,1-Propanetriyl-tris-(mercaptoethanat), 1,1,1-Propanetriyl-tris-(mercaptoproprionat), 1,1,1-Propanetriyl-tris-(mercaptobutanat), 2-hydroxmethyl-2-methyl-1,3-propandiol-tris-(mercaptoacetat), 2-hydroxmethyl-2-methyl-1,3-propandiol-tris-(mercaptoethanat), 2-hydroxmethyl-2-methyl-1,3-propandiol-tris-(mercaptopropionat), 2-hydroxmethyl-2-methyl-1,3-propandiol-tris-(mercaptobutanat).

Besonders bevorzugte trifunktionale Regler sind Glycerylthioglycolat, Trimethylolpropan-tris(2-mercaptoacetat), 2-hydroxmethyl-2-methyl-1,3-propandiol tris-(mercaptoacetat).

Bevorzugte tetrafunktionale Mercaptane sind Pentaerythrit-tetrakis-(2-mercaptoacetat), Pentaerythrit-tetrakis-(2-mercaptoethanat), Pentaerythrit-tetrakis(3-mercaptopropionat), Pentaerythrit-tetrakis-(4-mercaptobutanat), Pentaerythrit-tetrakis(5-mercaptopentanat), Pentaerythrit-tetrakis-(6-mercaptohexanat).

Als weitere polyfunktionale Regler eignen sich polyfunktionale Regler Si-Verbindungen der Formeln 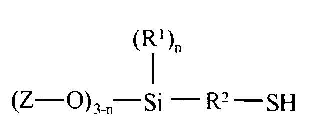 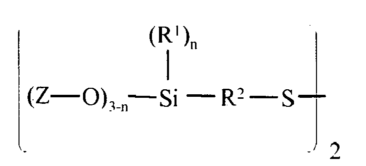 in der
- n: ein Wert von 0 bis 2 ist,
- R¹: eine C₁-C₁₆-Alkylgruppe oder Phenylgruppe bedeutet,
- R²: eine C₁-C₁₈-Alkylgruppe, die Cyclohexyl- oder Phenylgruppe bezeichnet,
- Z: für eine C₁-C₁₈ Alkylgruppe, C₂-C₁₈-Alkylengruppe oder C₂-C₁₈-Alkinylgruppe steht, deren Kohlenstoffatome durch nicht benachbarte Sauerstoff- oder Halogenatome ersetzt sein können, oder für eine der Gruppen 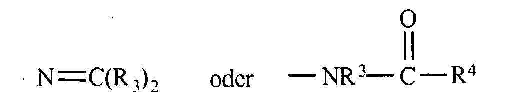
in denen
- R₃/R³: eine C₁-C₁₂-Alkylgruppe bedeutet und
- R⁴: eine C₁-C₁₈-Alkylgruppe bezeichnet.

Alle genannten Regler können einzeln oder in Kombination miteinander eingesetzt werden, wobei Mercaptoethanol alleine oder in Mischungen besonders bevorzugt ist.

### Vernetzer

In einer Ausführungsform der Erfindung werden Herstellung der Polymere b) Vernetzer verwendet. Die Bedeutung des Begriffs "Vernetzer" ist dem Fachmann bekannt. Der Vernetzer ist bevorzugt ausgewählt aus Verbindungen mit wenigstens 2 ethylenisch ungesättigten, nichtkonjugierten, zur radikalischen Copolymerisation geeigneten Doppelbindungen pro Molekül.

Geeignete Vernetzer sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Vernetzer enthalten aber mindestens zwei ethylenisch ungesättigte Gruppen.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000.

Außer den Homopolymeren des Ethylenoxids bzw. Propylenoxids können auch Blockcopolymere aus Ethylenoxid oder Propylenoxid oder Copolymere, die Ethylenoxid- und Propylenoxid-Gruppen eingebaut enthalten, eingesetzt werden.

Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Bevorzugte mehrwertige Alkohole in diesem Zusammenhang sind auch Di- und Trisaccaride.

Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer sind die Vinylester oder die Ester einwertiger, ungesättigter Alkohole mit ethylenisch ungesättigten C₃- bis C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Geeignet als Vernetzer sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Als Vernetzer sind ferner geeignet die Amide von (Meth)Acrylsäure, Itaconsäure und Maleinsäure sowie N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z.B. Triallylmethylammoniumchlorid oder -methylsulfat, als Vernetzer geeignet.

Geeignet sind auch N-Vinyl- oder N-Allyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z.B. N,N'-Divinylethylenharnstoff, N,N'-Divinylpropylenharnstoff oder N,N'-Diallylharnstoff.

Geeignet sind auch Alkylenbisacrylamide wie Methylenbisacrylamid und N,N'-(2,2)butan und 1,1'-bis-(3,3'-vinylbenzimidazolith-2-on)-1,4-butan.

Andere geeignete Vernetzer sind beispielsweise Alkylenglykoldi(meth)acrylate wie Ethylenglykoldiacrylat, Ethylenglykoldimethacrylat, Tetraethlyenglykolacrylat, Tetraethylenglykoldimethacrylat, Diethylenglykolacrylat, Diethylenglykolmethacrylat, Vinylacrylat, Allylacrylat, Allylmethacrylat, Divinyldioxan, Pentaerythritallylether sowie Gemische dieser Vernetzer.

Weitere geeignete Vernetzer sind Divinyldioxan, Tetraallylsilan oder Tetravinylsilan.

Besonders bevorzugt eingesetzte Vernetzer sind Methylenbisacrylamid, Triallylamin, Triallylalkylammoniumsalze, Divinylimidazol, Pentaerythrittriallylether, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Vernetzer sind Pentaerythrittriallylether, Methylenbisacrylamid, N,N'-Divinylethylenharnstoff, Triallylamin und Triallylmonoalkylammoniumsalze, und Acrylsäureester von Ethylenglykol, Butandiol, Trimethylolpropan oder Glycerin oder Acrylsäureester von mit Ethylenoxid und/oder Epichlorhydrin umgesetzten Glykol, Butandiol, Trimethylolpropan oder Glycerin. Am meisten bevorzugt ist Pentaerythrittriallylether.

Selbstverständlich können auch Mischungen der vorgenannten Verbindungen eingesetzt werden. Der Vernetzer ist vorzugsweise im Reaktionsmedium löslich. Ist die Löslichkeit des Vernetzers im Reaktionsmedium gering, so kann er in einem Monomeren oder in einer Monomerenmischung gelöst werden oder aber in einem Lösungsmittel gelöst zudosiert werden, das sich mit dem Reaktionsmedium mischt. Besonders bevorzugt sind solche Vernetzer, die in der Monomermischung löslich sind.

Wenn Vernetzer zur Polymerisation eingesetzt werden, dann in Mengen von wenigstens 0,01, bevorzugt wenigstens 0,05, besonders bevorzugt wenigstens 0,1 und höchstens 5, bevorzugt höchstens 2 und besonders bevorzugt höchstens 1 Gew.-%, bezogen auf die Gesamtmenge der zu polymerisierenden Monomere.

In einer besonders bevorzugten Ausführungsform der Erfindung wird Pentaerythrittriallylether in einer Menge von 0,1 Gew.-% bis 0,7 Gew.-%, am meisten bevorzugt in einer Menge von 0,3 Gew.-% bis 0,6 Gew.% eingesetzt.

Die Gew.-%-Menge des Vernetzers bezieht sich auf die zur Herstellung des Polymers verwendete Gesamtmenge der Monomeren.

Die nach der Polymerisation vorliegenden Reaktionslösungen bzw. -dispersionen sind bei 50°C über einen Zeitraum von wenigstens 1 Woche, bevorzugt wenigstens 1 Monat, besonders bevorzugt wenigstens 3 Monaten, weiter bevorzugt wenigstens 6 Monaten und insbesondere wenigstens 12 Monaten stabil, d.h. es findet im wesentlichen keine weitergehende Phasenseparation statt.

Die Herstellung von Polymer b2) erfolgt in der Regel dergestalt, dass zur Polymerisation > 20 Gew.-%, oft > 25 Gew.-% und häufig > 30 Gew.-% der zu verwendenden Monomere bezogen auf die Gesamtmenge der resultierenden wässrigen PolymerDispersion eingesetzt werden.

Wesentlich ist, dass die Gesamtmenge der zu verwendenden Monomer bis zu einem Umsatz von > 90 Gew.-%, oft > 95 Gew.-% oder häufig > 98 Gew.-% polymerisiert wird.

Die Herstellung von Polymer b2) kann entweder nach der sogenannten Batchfahrweise unter Vorlage der Gesamtmenge der zu verwendenden Monomere oder nach der sogenannten Zulauffahrweise erfolgen.

Erfolgt die Polymerisation in Batchfahrweise, erfolgt die Vorlage aller Komponenten bis auf den Radikalinitiator im Polymerisationsreaktor. Anschließend wird das wässrige Polymerisationsgemisch unter Rühren auf Polymerisationstemperatur aufgeheizt und danach der Radikalinitiator diskontinuierlich oder kontinuierlich zugegeben.

In einer bevorzugten Ausführungsform wird das erfindungsgemäße Verfahren mittels Zulauffahrweise durchgeführt. Dabei werden einzelne oder alle Reaktionskomponenten ganz oder teilweise, absatzweise oder kontinuierlich, gemeinsam oder in getrennten Zuläufen dem wässrigen Reaktionsmedium zudosiert.

Vorteilhaft wird wenigstens eine Teilmenge des wenigstens einen Salzes und von Polymer a) sowie gegebenenfalls eine Teilmenge des wenigstens einen Radikalinitiators und/oder der zu verwendenden Monomere im wässrigen Reaktionsmedium unter Rühren vorgelegt und unter Polymerisationsbedingungen die gegebenenfalls verbliebenen Restmengen des wenigstens einen organischen oder anorganischen Salzes und Polymer a) sowie die Gesamtmenge bzw. gegebenenfalls verbliebene Restmenge des wenigstens einen Radikalinitiators und/oder der zu verwendenden Monomere diskontinuierlich oder insbesondere kontinuierlich zudosiert.

Die bei der Polymerisation erhaltenen Wasser-in-Wasser-Dispersionen können im Anschluss an den Polymerisationsprozess einer physikalischen oder chemischen Nachbehandlung unterworfen werden.

### Kosmetische Zusammensetzungen

Die erfindungsgemäßen kosmetischen Zusammensetzungen können als wässrige oder wässrig-alkoholische Lösungen, O/W- sowie W/O-Emulsionen, Hydrodispersionsformulierungen, feststoffstablilisierte Formulierungen, Stiftformulierungen, PIT-Formulierungen, in Form von Cremes, Schäumen, Sprays (Pumpspray oder Aerosol), Gelen, Gelsprays, Lotionen, Ölen, Ölgelen oder Mousse vorliegen und dementsprechend mit üblichen weiteren Hilfsstoffen formuliert werden.

Bevorzugte kosmetische Zusammensetzungen im Sinne der vorliegenden Erfindung sind Shampoos und Haarpflegemittel. Die Erfindung betrifft demnach auch Zusammensetzungen zur Reinigung und/oder Pflege der Haare.

Insbesondere betrifft die Erfindung Haarpflegemittel ausgewählt aus der Gruppe bestehend aus Vorbehandlungsmitteln, Haarspülungen, Haarconditionern, Haarbalsamen, leave-on-Haarkuren, rinse-off-Haarkuren, Haarwässern, Pomaden, Frisiercremes, Frisierlotionen, Frisiergelen, Spitzenfluids, Hot-Oil-Treatments und Schaumkuren.

Weiterhin betrifft die Erfindung kosmetische Zusammensetzungen, die ausgewählt sind aus Gelcremes, Hydroformulierungen, Stiftformulierungen, kosmetischen Ölen und Ölgelen, Mascara, Selbstbräunern, Gesichtspflegemitteln, Körperpflegemitteln, After-Sun-Präparaten, Haarverformungsmitteln und Haarfestigern.

Weitere erfindungsgemäße kosmetische Zusammensetzungen sind hautkosmetische Zusammensetzungen, insbesondere solche zur Pflege der Haut. Diese liegen insbesondere als W/O- oder O/W-Hautcremes, Tag- und Nachtcremes, Augencremes, Gesichtscremes, Antifaltencremes, Mimikcremes, Feuchthaltecremes, Bleichcremes, Vitamincremes, Hautlotionen, Pflegelotionen und Feuchthaltelotionen vor.

Weiterhin eignen sich die erfindungsgemäßen Polymerkombinationen als Inhaltsstoffe für hautkosmetische Zubereitungen wie Gesichtswässer, Gesichtsmasken, Deodorantien und andere kosmetische Lotionen und für die Verwendung in der dekorativen Kosmetik, beispielsweise als Abdeckstift, Theaterfarbe, in Mascara und Lidschatten, Lippenstiften, Kajalstiften, Eyelinern, Makeup, Grundierungen, Rouges und Pudern und Augenbrauenstiften.

Außerdem können die erfindungsgemäßen Zusammensetzungen verwendet werden in Nose-Strips zur Porenreinigung, in Antiaknemitteln, Repellentien, Rasiermitteln, Haarentfernungsmitteln, Intimpflegemitteln, Fußpflegemitteln sowie in der Babypflege.

Die Zusammensetzungen enthalten neben den erfindungsgemäß erhältlichen Polymer-Kombinationen weitere kosmetisch akzeptable Zusätze wie beispielsweise Emulgatoren und Co-Emulgatoren, Lösungsmittel, Tenside, Ölkörper, Konservierungsmittel, Parfümöle, kosmetische Pflege- und Wirkstoffe wie AHA-Säuren, Fruchtsäuren, Ceramide, Phytantriol, Collagen, Vitamine und Provitamine, beispielsweise Vitamin A, E und C, Retinol, Bisabolol, Panthenol, natürliche und synthetische Lichtschutzmittel, Naturstoffe, Trübungsmittel, Lösungsvermittler, Repellentien, Bleichmittel, Färbemittel, Tönungsmittel, Bräunungsmittel (z.B. Dihydroxyaceton), Mikropigmente wie Titanoxid oder Zinkoxid, Überfettungsmittel, Perlglanzwachse, Konsistenzgeber, Verdicker, Solubilisatoren, Komplexbildner, Fette, Wachse, Silikonverbindungen, Hydrotrope, Farbstoffe, Stabilisatoren, pH-Wert Regulatoren, Reflektoren, Proteine und Proteinhydrolysate (z.B. Weizen-, Mandel- oder Erbsenproteine), Ceramid, Eiweißhydrolysate, Salze, Gelbildner, Konsistenzgeber, Silikone, Feuchthaltemittel (z.B. 1,2-Pentandiol), Rückfetter, UV-Lichtschutzfilter und weitere übliche Additive. Des weiteren können zur Einstellung der jeweils gewünschten Eigenschaften insbesondere auch weitere Polymere enthalten sein.

Weitere erfindungsgemäße kosmetische Zusammensetzungen sind Körperpflegezusammensetzungen sowie Wasch-, Dusch- und Badepräparate.

Unter Wasch-, Dusch- und Badepräparaten werden im Rahmen dieser Erfindung Seifen von flüssiger bis gelförmiger Konsistenz, wie Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und -cremes verstanden.

Geeignete weitere Inhaltsstoffe für diese erfindungsgemäßen Wasch-, Dusch- und Badepräparate sind im Folgenden beschrieben.

Die erfindungsgemäßen Zusammensetzungen enthalten neben den Polymerkombinationen weitere kosmetisch akzeptable Zusätze wie beispielsweise Emulgatoren und Co-Emulgatoren, Lösungsmittel, Tenside, Ölkörper, Konservierungsmittel, Parfümöle, kosmetische Pflege- und Wirkstoffe wie AHA-Säuren, Fruchtsäuren, Ceramide, Phytantriol, Collagen, Vitamine und Provitamine, beispielsweise Vitamin A, E und C, Retinol, Bisabolol, Panthenol, natürliche und synthetische Lichtschutzmittel, Naturstoffe, Trübungsmittel, Lösungsvermittler, Repellentien, Bleichmittel, Färbemittel, Tönungsmittel, Bräunungsmittel (z.B. Dihydroxyaceton), Mikropigmente wie Titanoxid oder Zinkoxid, Überfettungsmittel, Perlglanzwachse, Konsistenzgeber, Verdicker, Solubilisatoren, Komplexbildner, Fette, Wachse, Silikonverbindungen, Hydrotrope, Farbstoffe, Stabilisatoren, pH-Wert Regulatoren, Reflektoren, Proteine und Proteinhydrolysate (z.B. Weizen-, Mandel- oder Erbsenproteine), Ceramid, Eiweißhydrolysate, Salze, Gelbildner, Konsistenzgeber, Silikone, Feuchthaltemittel (z.B. 1,2-Pentandiol), Rückfetter, UV-Lichtschutzfilter und weitere übliche Additive. Des weiteren können zur Einstellung der jeweils gewünschten Eigenschaften insbesondere auch weitere Polymere enthalten sein.

Die erfindungsgemäßen kosmetischen Zusammensetzungen enthalten die erfindungsgemäßen Polymerkombinationen in einer Menge von 0,01 bis 20 Gew.-%, bevorzugt von 0,05 bis 5 Gew.-%, besonders bevorzugt 0,1 bis 1,5 Gew.-%, bezogen auf das Gewicht der Zusammensetzung.

In einer bevorzugten Ausführungsform der Erfindung enthalten die erfindungsgemäßen Körperpflegezusammensetzungen, Wasch-, Dusch- und Badepräparate sowie Shampoos und Haarpflegemittel weiterhin wenigstens ein Tensid.

In einer weiteren bevorzugten Ausführungsform der Erfindung enthalten die erfindungsgemäßen Körperpflegezusammensetzungen, Wasch-, Dusch- und Badepräparate, Shampoos und Haarpflegemittel neben den Polymeren weiterhin wenigstens eine Öl- und/oder Fettphase und ein Tensid.

### Tenside

Als Tenside können anionische, kationische, nichtionische und/oder amphotere Tenside eingesetzt werden.

Vorteilhafte waschaktive anionische Tenside im Sinne der vorliegenden Erfindung sind
- Acylaminosäuren und deren Salze, wie Acylglutamate, insbesondere Natriumacylglutamat
- Sarcosinate, beispielsweise Myristoyl Sarcosin, TEA-Lauroyl Sarcosinat, Natriumlauroylsarcosinat und Natriumcocoylsarcosinat,
Sulfonsäuren und deren Salze, wie
- Acylisethionate, beispielsweise Natrium- oder Ammoniumcocoylisethionat
- Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat, Dinatrium PEG-5 Laurylcitratsulfosuccinat und Derivate,
- Alkylethersulfate, beispielsweise Natrium-, Ammonium-, Magnesium-, MIPA-, TIPA-Laurethsulfat, Natriummyrethsulfat und Natrium C₁₂₋₁₃ Parethsulfat,
- Alkylsulfate, beispielsweise Natrium-, Ammonium- und TEA-Laurylsulfat.
   Weitere vorteilhafte anionische Tenside sind
- Taurate, beispielsweise Natriumlauroyltaurat und Natriummethylcocoyltaurat,
- Ether-Carbonsäuren, beispielsweise Natriumlaureth-13 Carboxylat und Natrium PEG-6 Cocamide Carboxylat, Natrium PEG-7-Olivenöl-Carboxylat
- Phosphorsäureester und Salze, wie beispielsweise DEA-Oleth-10 Phosphat und Dilaureth-4 Phosphat,
- Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat,
- Acylglutamate wie Di-TEA-palmitoylaspartat und Natrium Caprylic/Capric Glutamat,
- Acylpeptide, beispielsweise Palmitoyl hydrolysiertes Milchprotein, Natrium Cocoyl hydrolysiertes Sojaprotein und Natrium-/Kalium Cocoyl hydrolysiertes Kollagen sowie Carbonsäuren und Derivate, wie beispielsweise Laurinsäure, Aluminiumstearat, Magnesiumalkanolat und Zinkundecylenat, Ester-Carbonsäuren, beispielsweise Calciumstearoyllactylat, Laureth-6 Citrat und Natrium PEG-4 Lauramidcarboxylat
- Alkylarylsulfonate.

Vorteilhafte waschaktive kationische Tenside im Sinne der vorliegenden Erfindung sind quaternäre Tenside. Quaternäre Tenside enthalten mindestens ein N-Atom, das mit 4 Alkyl- oder Arylgruppen kovalent verbunden ist. Vorteilhaft sind beispielsweise Alkylbetain, Alkylamidopropylbetain und Alkylamidopropylhydroxysultain.

Weitere vorteilhafte kationische Tenside im Sinne der vorliegenden Erfindung sind ferner
- Alkylamine,
- Alkylimidazole und
- ethoxylierte Amine
und insbesondere deren Salze.

Vorteilhafte waschaktive amphotere Tenside im Sinne der vorliegenden Erfindung sind Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat, Natriumacylamphopropionat, und N-Kokosfettsäureamidoethyl-N-hydroxyethylglycinat Natriumsalze.

Weitere vorteilhafte amphotere Tenside sind N-Alkylaminosäuren, beispielsweise Aminopropylalkylglutamid, Alkylaminopropionsäure, Natriumalkylimidodipropionat und Lauroamphocarboxyglycinat.

Vorteilhafte waschaktive nicht-ionische Tenside im Sinne der vorliegenden Erfindung sind
- Alkanolamide, wie Cocamide MEA/DEA/MIPA,
- Ester, die durch Veresterung von Carbonsäuren mit Ethylenoxid, Glycerin, Sorbitan oder anderen Alkoholen entstehen,
- Ether, beispielsweise ethoxylierte Alkohole, ethoxyliertes Lanolin, ethoxylierte Polysiloxane, propoxylierte POE Ether, Alkylpolyglycoside wie Laurylglucosid, Decylglycosid und Cocoglycosid, Glycoside mit einem HLB-Wert von wenigstens 20 (z.B. Belsil^{®}SPG 128V (Wacker)).

Weitere vorteilhafte nicht-ionische Tenside sind Alkohole und Aminoxide, wie Cocoamidopropylamin-Oxid.

Bevorzugte anionische, amphotere und nichtionische Shampoo-Tenside sind bleispielsweise in "Kosmetik und Hygiene von Kopf bis Fuß", Hrsg. W. Umbach, 3. Auflage, Wiley-VCH, 2004, S.131-134 genannt, worauf an dieser Stelle in vollem Umfang Bezug genommen wird.

Unter den Alkylethersulfaten sind insbesondere Natriumalkylethersulfate auf Basis von zwei- oder dreifach ethoxyliertem Lauryl- und Myristylalkohol bevorzugt. Sie übertreffen die Alkylsulfate deutlich bezüglich der Unempfindlichkeit gegen Wasserhärte, der Verdickbarkeit, der Kältelöslichkeit und insbesondere der Haut- und Schleimhautverträglichkeit. Sie können auch als alleinige Waschrohstoffe für Shampoos eingesetzt werden können. Laurylethersulfat weist bessere Schaumeigenschaften als Myristylethersulfat auf, ist diesem aber in der Milde unterlegen.

Alkylethercarboxylate mit mittlerem und besonders mit höherem gehören zu den mildesten Tensiden überhaupt, zeigen aber ein schlechtes Schaum- und Viskositätsverhalten. Sie werden oft in Kombination mit Alkylethersulfaten und amphoteren Tensiden in Haarwaschmitteln eingesetzt.

Sulfobemsteinsäureester (Sulfosuccinate) sind mild und gut schäumende Tenside werden aber wegen ihrer schlechten Verdickbarkeit bevorzugt nur zusammen mit anderen anionischen und amphoteren Tensiden und wegen ihrer geringen Hydrolysestabilität bevorzugt nur in neutralen bzw. gut gepufferten Produkten eingesetzt. Arnidopropylbetaine sind als alleinige Waschrohstoffe praktisch unbedeutend, da ihr Schaumverhalten sowie ihre Verdickbarkeit nur mäßig ausgeprägt sind. Demgegenüber weisen diese Tenside eine ausgezeichnete Haut- und Augenschleimhautverträglichkeit auf. In Kombination mit anionischen Tensiden lässt sich deren Milde synergistisch verbessern. Bevorzugt ist die Verwendung von Cocamidopropylbetain. Arnphoacetate/Arnphodiacetate besitzen als amphotere Tenside eine sehr gute Haut- und Schleimhautverträglichkeit und können haarkonditionierend wirken bzw. die Pflegewirkung von Zusatzstoffen erhöhen. Sie werden ähnlich wie die Betaine zur Optimierung von Alkylethersulfat-Formulierungen eingesetzt. Am meisten bevorzugt sind Natriumcocoamphoacetat und Dinatriumcocoamphodiacetat.

Alkylpolyglykoside sind nichtionische Waschrohstoffe. Sie sind mild, haben gute Universaleigenschaften, schäumen jedoch schwach. Aus diesem Grund werden sie bevorzugt in Kombinationen mit anionischen Tensiden verwendet.

Sorbitanester gehören ebenfalls zu den nichtionischen Waschrohstoffen. Wegen ihrer ausgezeichneten Milde werden sie bevorzugt zum Einsatz bei Baby-Shampoos verwendet. Als Schwachschäumer werden sie bevorzugt in Kombination mit anionischen Tensiden eingesetzt.

Es ist vorteilhaft, das oder die waschaktiven Tenside aus der Gruppe der Tenside zu wählen, welche einen HLB-Wert von mehr als 25 haben, besonders vorteilhaft sind solche, welchen einen HLB-Wert von mehr als 35 haben.

Es ist erfindungsgemäß von Vorteil, wenn ein oder mehrere dieser Tenside in einer Konzentration von 1 bis 30 Gewichts-%, bevorzugt in einer Konzentration 5 von 25 bis Gewichts-% und ganz besonders bevorzugt in einer Konzentration von 10 bis 20 Gewichts-%, jeweils bezogen auf das Gesamtgewicht der Zusammensetzung eingesetzt werden.

### Polysorbate

Als waschaktive Agentien können auch Polysorbate vorteilhaft in die erfindungsgemäßen Zusammensetzungen eingearbeitet werden.

Im Sinne der Erfindung vorteilhafte Polysorbate sind beispielsweise
- Polyoxyethylen(20)sorbitanmonolaurat (Tween^{®}20, CAS-Nr. 9005-64-5)
- Polyoxyethylen(4)sorbitanmonolaurat (Tween^{®}21, CAS-Nr. 9005-64-5)
- Polyoxyethylen(4)sorbitanmonostearat (Tween^{®}61, CAS-Nr. 9005-67-8)
- Polyoxyethylen(20)sorbitantristearat (Tween^{®}65, CAS-Nr. 9005-71 -4)
- Polyoxyethylen(20)sorbitanmonooleat (Tween^{®}80, CAS-Nr. 9005-65-6)
- Polyoxyethylen(5)sorbitanmonooleat (Tween^{®}81, CAS-Nr. 9005-65-5)
- Polyoxyethylen(20)sorbitantrioleat (Tween^{®}85, CAS-Nr. 9005-70-3).

Besonders vorteilhaft sind
- Polyoxyethylen(20)sorbitanmonopalmitat (Tween^{®}40, CAS-Nr. 9005-66-7) und
- Polyoxyethylen(20)sorbitanmonostearat (Tween^{®}60, CAS-Nr. 9005-67-8).

Die Polysorbate werden vorteilhaft in einer Konzentration von 0,1 bis 5 und insbesondere in einer Konzentration von 1,5 bis 2,5 Gew.-%, bezogen auf das Gesamtgewicht der Zusammensetzung einzeln oder als Mischung mehrer Polysorbate, eingesetzt.

### Konditionierungsmittel

Sofern gewünscht, werden als Konditionierungsmittel für die erfindungsgemäßen kosmetischen Zusammensetzungen bevorzugt diejenigen Konditionierungsmittel gewählt, die auf Seite 34, Zeile 24 bis Seite 37, Zeile 10 der WO 2006/106140 beschrieben sind. Auf den Inhalt der genannten Textstelle wird hiermit in vollem Umfang Bezug genommen.

Die erfindungsgemäßen Zusammensetzungen sind geeignet, die Abscheidungsmenge und -geschwindigkeit sowie die Verweilzeit von in diesen erfindungsgemäßen Zusammensetzungen gegebenenfalls ebenfalls ent-haltenen weiteren Wirkstoffen, wie beispielsweise Silikonen oder UV-Lichtschutzfiltern, auf der Haut und/oder dem Haar zu verstärken bzw. zu erhöhen. Substanzen oder Mittel, die solche Effekte besitzen, werden auch als Depositioning Aids bezeichnet.

US 6,998,113 beschreibt rinse-off-Zubereitungen, die dazu führen, dass die damit behandelte Haut effektiv vor UV-Strahlung geschützt wird. Einige der dort beschriebenen Zubereitungen enthalten kationische Polymere. Im Sinne der vorliegenden Erfindung können auch die erfindungsgemäßen Kombinationen aus Polymer a) und Polymer b) in den Zubereitungen der US 6,998,113 verwendet werden. Insbesondere können die erfindungsgemäßen Kombinationen für den von der US 6,998,113 genannten Zweck in Sonnenschutz, Wasch- und Badepräparaten eingesetzt werden. Auf die Offenbarung der US 6,998,113 wird hiermit in vollem Umfang Bezug genommen.

Geeignete Silikone sind beispielsweise in der US 5,935,561, Spalte 13, Z.64 bis Spalte 18, Z.61 dargestellt, worauf hiermit in vollem Umfang Bezug genommen wird. Stellvertretend seien genannt:
- Dimethicone
- Polyalkyl -oder Polyarylsiloxane (US 5,935,561, Spalte 13, Formel I)
- Alkylamino substituierte Silikone (US 5,935,561, Spalte 14, Formel II (Amodimethicone))
- Kationische Silikone (US 5,935,561, Spalten 14 und 15, Formel III)
- Trimethylsilylamodimethicone (US 5,935,561, Spalte 15, Formel IV)
- Silikone gemäß US 5,935,561, Spalte 15, Formel V
- cyclische Polysiloxane gemäß US 5,935,561, Spalte 16, Formel VI

### Rheologiemodifizierungsmittel

Bei den geeigneten Rheologiemodifizierungsmitteln handelt es sich vor allem um Verdicker.

Für Shampoos und Haarpflegemittel geeignete Verdicker sind in "Kosmetik und Hygiene von Kopf bis Fuß", Hrsg. W. Umbach, 3. Auflage, Wiley-VCH, 2004, S.235-236 genannt, worauf an dieser Stelle vollumfänglich Bezug genommen wird.

Geeignete Verdickungsmittel für die erfindungsgemäßen kosmetischen Zusammensetzungen sind beispielsweise auf Seite 37, Zeile 12 bis Seite 38, Zeile 8 der WO 2006/106140 beschrieben. Auf den Inhalt der genannten Textstelle wird hiermit in vollem Umfang Bezug genommen.

Besonders bevorzugt sind erfindungsgemäße kosmetische Zusammensetzungen, die Polyurethane als sogenannte Assoziativverdicker enthalten. Derartige Polyurethane sind beispielsweise beschrieben in EP 1584331,EP 1013264, WO 2006/002813, EP 1241198, WO 02/083093, WO 02/088212, WO 02/044236, EP 725097, US 4,079,028, EP 618243. Insbesondere bevorzugt sind erfindungsgemäße kosmetische Zusammensetzungen, die Polyurethane wie in EP 1584331, Absätze [0009] bis [0016] beschrieben, enthalten. Auf die Offenbarungen der vorgenannten Schriften wird hiermit in vollem Umfang Bezug genommen.

### Konservierungsmittel

Die erfindungsgemäßen kosmetischen Zusammensetzungen können auch Konservierungsmittel enthalten. Zusammensetzungen mit hohen Wassergehalten müssen zuverlässig vor Verkeimung geschützt sein. Geeignete Konservierungsmittel für die erfindungsgemäßen kosmetischen Zusammensetzungen sind beispielsweise auf Seite 38, Zeile 10 bis Seite 39, Zeile 18 der WO 2006/106140 beschrieben. Auf den Inhalt der genannten Textstelle wird hiermit in vollem Umfang Bezug genommen.

Komplexbildner: Da die Rohstoffe und auch die Shampoos selbst überwiegend in Stahlapparaturen hergestellt werden, können die Endprodukte Eisen(-Ionen) in Spurenmengen enthalten. Um zu verhindern, dass diese Verunreinigungen über Reaktionen mit Farbstoffen und Parfümölbestandteilen die Produktqualität nachteilig beeinflussen, werden Komplexbildner wie Salze der Ethylendiamintetraessigsäure, der Nitrilotriessigsäure, der Iminodibernsteinsäure oder Phosphate zugesetzt.

UV- Lichtschutzfilter: Um die in den erfindungsgemäßen Zusammensetzungen enthaltenen Inhaltsstoffe wie beispielsweise Farbstoffe und Parfümöle gegen Veränderungen durch UV-Licht zu stabilisieren, können UV- Lichtschutzfilter, wie z. B. Benzophenon-Derivate, eingearbeitet werden. Geeignete UV- Lichtschutzfilter für die erfindungsgemäßen kosmetischen Zusammensetzungen sind beispielsweise auf Seite 39, Zeile 20 bis Seite 41 Zeile 10 der WO 2006/106140 beschrieben. Auf den Inhalt der genannten Textstelle wird hiermit in vollem Umfang Bezug genommen.

Antioxidantien: Ein Gehalt der erfindungsgemäßen Zusammensetzungen an Antioxidantien ist im allgemeinen bevorzugt. Erfindungsgemäß können als Antioxidantien alle für kosmetische Anwendungen geeigneten oder gebräuchlichen Antioxidantien verwendet werden. Geeignete Antioxidantien für die erfindungsgemäßen kosmetischen Zusammensetzungen sind beispielsweise auf Seite 41, Zeile 12 bis Seite 42 Zeile 33 der WO 2006/106140 beschrieben. Auf den Inhalt der genannten Textstelle wird hiermit in vollem Umfang Bezug genommen.

Puffer: Puffer gewährleisten die pH-Wert-Stabilität der Zusammensetzungen. Überwiegend verwendet werden Citrat-, Lactat- und Phosphat-Puffer.

Lösungsvermittler: Sie werden eingesetzt, um pflegende Öle oder Parfümöle klar in Lösung zu bringen und auch in der Kälte klar in Lösung zu halten. Die gängigsten Lösungsvermittler sind ethoxylierte nichtionische Tenside, z. B. hydrierte und ethoxylierte Ricinusöle.

Keimhemmende Mittel: Weiterhin können auch keimhemmende Mittel eingesetzt werden. Dazu gehören generell alle geeigneten Konservierungsmittel mit spezifischer Wirkung gegen grampositive Bakterien, z.B. Triclosan (2,4,4'-Trichlor-2'-hydroxydiphenylether), Chlorhexidin (1,1'-Hexamethylenbis[5-(4-chlorphenyl)-biguanid) sowie TTC (3,4,4'-Trichlorcarbanilid). Quartäre Ammonium-Verbindungen sind prinzipiell ebenfalls geeignet und werden bevorzugt für desinfizierende Seifen und Waschlotionen verwendet. Auch zahlreiche Riechstoffe haben antimikrobielle Eigenschaften. Auch eine große Anzahl etherischer Öle bzw. deren charakteristische Inhaltsstoffe wie z.B. Nelkenöl (Eugenol), Minzöl (Menthol) oder Thymianöl (Thymol), zeigen eine ausgeprägte antimikrobielle Wirksamkeit.

Die antibakteriell wirksamen Stoffe werden in der Regel in Konzentrationen von ca. 0,1 bis 0,3 Gew.-% eingesetzt.

Dispergiermittel: Wenn in den erfindungsgemäßen Zusammensetzungen unlösliche Wirkstoffe, z.B. Antischuppenwirkstoffe oder Siliconöle, dispergiert und auf Dauer in Schwebe gehalten werden sollen, müssen Dispergiermittel und Verdicker wie z. B. Magnesium-Aluminium-Silicate, Bentonite, Fettacyl-Derivate, Polyvinylpyrrolidon oder Hydrokolloide, z. B. Xanthan Gum oder Carbomere, eingesetzt werden. Erfindungsgemäß sind Konservierungsmittel in einer Gesamtkonzentration von höchstens 2, bevorzugt höchstens 1,5 und besonders bevorzugt höchstens 1 Gewichts-%, bezogen auf das Gesamtgewicht der Zusammensetzung, enthalten.

### Öle, Fette und Wachse

Die erfindungsgemäßen Zusammensetzungen enthalten bevorzugt Öle, Fette und/oder Wachse.

Vorteilhafterweise werden als Inhaltsstoffe für die erfindungsgemäßen Zusammensetzungen diejenigen Öle, Fette und/oder Wachse gewählt, die auf Seite 28, Zeile 39 bis Seite 34, Zeile 22 der WO 2006/106140 beschrieben sind. Auf den Inhalt der genannten Textstelle wird hiermit in vollem Umfang Bezug genommen.

Der Gehalt an weiteren Ölen, Fetten und Wachsen beträgt höchstens 50, bevorzugt 30, weiter bevorzugt höchstens 20 Gewichts-%, bezogen auf das Gesamtgewicht der Zusammensetzung.

Die Zusammensetzungen können außer den vorgenannten Substanzen gegebenenfalls die in der Kosmetik üblichen Zusatzstoffe, beispielsweise Parfüm, Farbstoffe, rückfettende Agentien, Komplexierungs- und Sequestrierungsagentien, Perlglanzagentien, Pflanzenextrakte, Vitamine, Wirkstoffe, Pigmente, die eine färbende Wirkung haben, weichmachende, anfeuchtende und/oder feuchthaltende Substanzen, oder andere übliche Bestandteile einer kosmetischen oder dermatologischen Formulierung wie Alkohole, Polyole, Polymere, organische Säuren zur pH-Wert-Einstellung, Schaumstabilisatoren, Elektrolyte, organische Lösemittel oder Silikonderivate enthalten.

Hinsichtlich der genannten, dem Fachmann bekannten weiteren Inhaltsstoffe für die Zusammensetzungen sei auf "Kosmetik und Hygiene von Kopf bis Fuß", Hrsg. W. Umbach, 3. Auflage, Wiley-VCH, 2004, S.123-128 verwiesen, worauf an dieser Stelle vollumfänglich Bezug genommen wird.

### Ethoxylierte Glycerin-Fettsäureester

Die erfindungsgemäßen Zusammensetzungen wie Körperflegemittel, Wasch- und Badepräparate sowie Shampoos und Haarpflegemittel enthalten gegebenenfalls ethoxylierte Öle ausgewählt aus der Gruppe der ethoxylierten Glycerin-Fettsäureester, insbesondere bevorzugt PEG-10 Olivenölglyceride, PEG-11 Avocadoölglyceride, PEG-11 Kakaobutterglyceride, PEG-13 Sonnenblumenölglyceride, PEG-15 Glycerylisostearat, PEG-9 Kokosfettsäureglyceride, PEG-54 Hydriertes Ricinusöl, PEG-7 Hydriertes Ricinusöl, PEG-60 Hydriertes Ricinusöl, Jojobaöl Ethoxylat (PEG-26 Jojoba-Fett-Säuren, PEG-26 Jojobaalkohol), Glycereth-5 Cocoat, PEG-9 Kokosfettsäureglyceride, PEG-7 Glycerylcocoat, PEG-45 Palmkemölglyceride, PEG-35 Ricinusöl, Olivenöl-PEG-7 Ester, PEG-6 Caprylisäure/Caprinsäureglyceride, PEG-10 Olivenölglyceride, PEG-13 Sonnenblumenölglyceride, PEG-7 Hydriertes Ricinusöl, Hydrierte Palmkernölglycerid-PEG-6 Ester, PEG-20 Maisölglyceride, PEG- 18 Glycerylolead-cocoat, PEG-40 Hydriertes Ricinusöl, PEG-40 Ricinusöl, PEG-60 Hydriertes Ricinusöl, PEG-60 Maisölglyceride, PEG-54 Hydriertes Ricinusöl, PEG-45 Palmkernölglyceride, PEG-80 Glycerylcocoat, PEG-60 Mandelölglyceride, PEG-60 "Evening Primrose" Glyceride, PEG-200 Hydriertes Glycerylpalmat, PEG-90 Glycerylisostearat.

Bevorzugte ethoxylierte Öle sind PEG-7 Glycerylcocoat, PEG-9 Kokosglyceride, PEG-40 Hydriertes Rizinusöl, PEG-200 hydriertes Glycerylpalmat.

Ethoxylierte Glycerin-Fettsäureester werden in wässrigen Reinigungsrezepturen zu verschiedenen Zwecken eingesetzt. Glycerin-Fettsäureester mit einem Ethoxylierungsgrad von ca. 30-50 dienen als Lösungsvermittler für unpolare Substanzen wie Parfumöle. Hochethoxylierte Glycerin-Fettsäureester werden als Verdicker eingesetzt.

### Wirkstoffe

In die erfindungsgemäßen Zusammensetzungen lassen sich verschiedenste Wirkstoffe mit unterschiedlicher Löslichkeit homogen einarbeiten. Vorteilhafte Wirkstoffe in den erfindungsgemäßen kosmetischen Zusammensetzungen sind beispielsweise auf Seite 44, Zeile 24 bis Seite 49 Zeile 39 der WO 2006/106140 beschrieben. Auf den Inhalt der genannten Textstelle wird hiermit in vollem Umfang Bezug genommen.

### UV-Lichtschutzmittel

Die erfindungsgemäßen Zusammensetzungen enthalten in einer bevorzugten Ausführungsform UV-Lichtschutzmittel zum Schutz der Haut und/oder der Haare. Geeignete UV-Lichtschutzmittel sind in der WO 2006/106114, S.24, Z.4 bis S.27, Z.27 ausführlich beschrieben, worauf hiermit in vollem Umfang Bezug genommen wird.

Vorteilhaft enthalten die Zusammensetzungen Substanzen, die UV-Strahlung im UVB-Bereich und Substanzen, die UV-Strahlung im UVA-Bereich absorbieren, wobei die Gesamtmenge der Filtersubstanzen z.B. 0,1 bis 30 Gew.-%, vorzugsweise 0,5 bis 20 Gew.-%, insbesondere 1 bis 15 Gew.-% beträgt, bezogen auf das Gesamtgewicht der Zusammensetzungen, um kosmetische Zusammensetzungen zur Verfügung zu stellen, welche die Haut vor dem gesamten Bereich der ultravioletten Strahlung schützen.

Der größte Teil der Lichtschutzmittel in den zum Schutz der menschlichen Epidermis dienenden kosmetischen oder dermatologischen Zusammensetzungen besteht aus Verbindungen, die UV-Licht im UV-B-Bereich absorbieren. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

### Perlglanzwachse

Geeignete Perlglanzwachse für die erfindungsgemäßen kosmetischen Zusammensetzungen sind beispielsweise auf Seite 50, Zeile 1 bis Zeile 16 der WO 2006/106140 beschrieben. Auf den Inhalt der genannten Textstelle wird hiermit in vollem Umfang Bezug genommen.

Die erfindungsgemäßen Zusammensetzungen können weiterhin Glitterstoffe und/oder andere Effektstoffe (z.B. Farbschlieren) enthalten.

### Emulgatoren

Die erfindungsgemäßen kosmetischen Zusammensetzungen liegen in einer bevorzugten Ausführungsform der Erfindung in Form von Emulsionen vor. Die Herstellung solcher Emulsionen erfolgt nach bekannten Methoden. Geeignete Emulgatoren für die erfindungsgemäßen Emulsionen sind beispielsweise auf Seite 50, Zeile 18 bis Seite 53, Zeile 4 der WO 2006/106140 beschrieben. Auf den Inhalt der genannten Textstelle wird hiermit in vollem Umfang Bezug genommen.

### Parfümöle

Sollen den erfindungsgemäßen kosmetischen Zusammensetzungen Parfumöle zugesetzt werden, so sind geeignete Parfümöle beispielsweise auf Seite 53, Zeile 10 bis Seite 54, Zeile 3 der WO 2006/106140 beschrieben. Auf den Inhalt der genannten Textstelle wird hiermit in vollem Umfang Bezug genommen.

### Pigmente

Gegebenenfalls enthalten die erfindungsgemäßen kosmetischen Zusammensetzungen weiterhin Pigmente. Die Pigmente liegen im Produkt meist in ungelöster Form vor und können in einer Menge von 0,01 bis 25 Gew.%, besonders bevorzugt von 5 bis 15 Gew.% enthalten sein. Die bevorzugte Teilchengröße beträgt 1 bis 200 µm, insbesondere 3 bis 150 µm, besonders bevorzugt 10 bis 100 µm.

Geeignete Pigmente für die erfindungsgemäßen Zusammensetzungen sind beispielsweise auf Seite 54, Zeile 5 bis Seite 55, Zeile 19 der WO 2006/106140 beschrieben. Auf den Inhalt der genannten Textstelle wird hiermit in vollem Umfang Bezug genommen.

### Polymere

Die erfindungsgemäßen kosmetischen Zusammensetzungen enthalten in einer bevorzugten Ausführungsform außer dem nach dem erfindungsgemäßen Verfahren hergestellten Polymer weitere Polymere.

Geeignete zusätzliche Polymere für die erfindungsgemäßen Zusammensetzungen sind beispielsweise auf Seite 55, Zeile 21 bis Seite 63, Zeile 2 der WO 2006/106140 beschrieben. Auf den Inhalt der genannten Textstelle wird hiermit in vollem Umfang Bezug genommen.

### Shampoo-Typen

An Shampoos werden je nach Haarqualität oder Kopfhautproblem gegebenfalls zusätzliche Anforderungen gestellt. Die Wirkungsweise der bevorzugten Shampoo-Typen mit den wichtigsten zusätzlichen Wirkungen bzw. wichtigsten speziellen Zielsetzungen wird nachfolgend beschrieben.

Erfindungsgemäß bevorzugt sind beispielsweise Shampoos für normales bzw. schnell fettendes bzw. geschädigtes Haar, Antischuppenshampoos, Babyshampoos und 2-in-1-Shampoos (Shampoo und Spülung in einem).

Shampoos für normales Haar: die Haarwäsche soll Haar und Kopfhaut von dem in Talgdrüsen gebildeten Hautfett, den aus Schweissdrüsen mit Wasser austretenden anorganischen Salzen, Aminosäuren, Harnstoff und Milchsäure, abgeschilferten Hautpartikeln, Umweltschmutz, Gerüchen und gegebenenfalls Rückständen haarkosmetischer Behandlungen befreien. Normales Haar bedeutet kurzes bis schulterlanges Haar, welches nur schwach geschädigt ist. Dementsprechend soll der Anteil an Konditionierhilfsstoffen auf diesen Haartyp optimiert sein.

Shampoos für schnell fettendes Haar: eine erhöhte Fettproduktion der Talgdrüsen der Kopfhaut führt bereits 1-2 Tage nach der Haarwäsche zu einer strähnigen, unansehnlichen Frisur. Öl- und wachsartige Hautfettbestandteile beschweren das Haar und mindern die Reibung von Haar zu Haar und verringern damit den Frisurenhalt. Das eigentliche haarkosmetische Problem bei schnell fettenden Haaren ist also das vorzeitige Zusammenfallen voluminöser Frisuren. Um dies zu vermeiden, muss man verhindern, dass die Haaroberfläche beschwert und zu glatt und geschmeidig wird. Dies wird bevorzugt durch die Tensidgrundlage aus gut reinigenden und besonders wenig substantiv ausgeprägten Waschrohstoffen erreicht. Zusätzliche Pflegestoffe, die sich zum Hautfett addieren würden, wie rückfettende Substanzen oder auch Konditionierhilfsstoffe, werden in Shampoos für schnell fettendes Haar nicht oder nur mit größter Vorsicht eingesetzt. Volumengebende Shampoos für feines Haar können vergleichbar formuliert werden.

Shampoos für trockenes, strapaziertes (geschädigtes) Haar. Die Struktur des Haares wird im Laufe des Haarwachstums durch mechanische Einflüsse wie Kämmen, Bürsten und vor allen Dingen Toupieren (Kämmen gegen die Wuchsrichtung), durch die Einwirkung von UV-Strahlung bzw. sichtbarem Licht und durch kosmetische Behandlungen, wie Dauerwellen, Blondierung oder Färbung verändert. Die Schuppenschicht der Haare weist von der Wurzel bis zur Spitze ein zunehmend strapazierteres Aussehen auf; in Extremfällen ist sie an der Spitze völlig abgetragen, und die Haarspitzen sind gespalten (Haarspliss). Geschädigtes Haar kann grundsätzlich nicht mehr in den Zustand des gesunden Haarnachwuchses zurückgeführt werden. Es gelingt aber, diesem Idealzustand hinsichtlich Griff, Glanz und Kämmbarkeit durch Verwendung von erfindungsgemäßen Shampoos mit gegebenfalls hohen Anteilen an pflegenden Stoffen (Konditioniermitteln) sehr nahe zu kommen.

Eine noch bessere Wirkung als mit einem Shampoo wird mit einem erfindungsgemäßen Haarpflegemittel beispielsweise in Form einer Spülungs- oder Kurmittelbehandlung nach der Haarwäsche erreicht.

Erfindungsgemäße 2-in-1- Shampoos sind besonders stark pflegende Shampoos, bei denen durch die Konzipierung als "Shampoo und Spülung in einem" der Zusatznutzen Pflege gleichwertig neben den Grundnutzen Reinigung gestellt wird. Erfindungsgemäße 2-in-1-Zusammensetzungen enthalten erhöhte Mengen ans Konditioniermitteln. Antischuppenshampoos: Erfindungsgemäße Antischuppenshampoos haben im Vergleich zu Antischuppenhaarwässern den Vorteil, dass sie nicht nur durch entsprechende Wirkstoffe gegen Schuppenbefall die Bildung neuer sichtbarer Schuppen verringern und bei langfristiger Anwendung verhindern, sondern mit der Haarwäsche auch bereits abgeschilferte Schuppen entfernen. Nach dem Ausspülen der Waschflotte bleibt allerdings nur ein geringer, jedoch ausreichender Anteil der Wirkstoffe auf Kopfhaut und Haar zurück. Es gibt verschiedene Antischuppen-Wirkstoffe, die in die erfindungsgemäßen Shampoo-Zusammensetzungen eingearbeitet werden können, wie z. B. Zinkpyrithion, Ketokonazol, Elubiol, Clotrimazol, Climbazol oder Pirocton Olamin. Zusätzlich weisen diese Substanzen eine die Abschilferung normalisierende Wirkung auf.

Die Grundlage von Antischuppenshampoos entspricht überwiegend der Formulierung von Shampoos für normales Haar mit gutem Reinigungseffekt.

Babyshampoos: bei einer bevorzugten Ausführungsform der Erfindung handelt es sich bei den erfindungsgemäßen Shampoo-Zubereitungen um Babyshampoos. Diese sind optimal haut- und schleimhautverträglich. Kombinationen von Waschrohstoffen mit sehr guter Hautverträglichkeit bilden die Basis dieser Shampoos. Zusätzliche Stoffe zur weiteren Verbesserung der Haut- und Schleimhautverträglichkeit und der Pflegeeigenschaften werden vorteilhaft zugefügt, wie z. B. nichtionische Tenside, Proteinhydrolysate und Panthenol oder Bisabolol. Alle notwendigen Rohstoffe und Hilfsstoffe, wie Konservierungsmittel, Parfümöle, Farbstoffe usw., werden unter dem Aspekt einer hohen Verträglichkeit und Milde ausgewählt werden.

Shampoos für trockene Kopfhaut: bei einer weiteren bevorzugten Ausführungsform der Erfindung handelt es sich bei den erfindungsgemäßen Shampoo-Zubereitungen um Shampoos für trockene Kopfhaut. Das primäre Ziel dieser Shampoos ist die Verhinderung der Austrocknung der Kopfhaut, da trockene Kopfhaut zu Juckreiz, Rötung und Entzündung führen kann. Wie auch bei den Babyshampoos bilden Kombinationen von Waschrohstoffen mit sehr guter Hautverträglichkeit die Basis dieser Shampoos. Zusätzlich können gegebenenfalls Rückfetter und Feuchthaltemittel, wie z. B. Glycerin oder Harnstoff, eingesetzt werden.

Die erfindungsgemäßen Shampoo-Zusammensetzungen können auch als Shampookonzentrate mit erhöhten Tensidgehalten von 20-30% vorliegen. Sie basieren auf speziellen Waschrohstoffkombinationen und Konsistenzregulatoren, die die gute Verteilbarkeit und das spontane Anschäumvermögen auch einer kleinen Anwendungsmenge gewährleisten. Ein besonderer Vorteil ist beispielsweise die Möglichkeit, die Ergiebigkeit von 200 ml Shampoo mit einer 100-ml-Flasche zu erreichen.

### Darreichung

Es ist vorteilhaft, wenn die erfindungsgemäßen Zusammensetzungen in einer Tube, einem Tiegel, einer Flasche oder Quetschflasche aufbewahrt und aus dieser heraus angewendet werden. Demzufolge sind auch Tuben, Tiegel, Flaschen oder Quetschflaschen, welche eine erfindungsgemäße Zusammensetzung enthalten, erfindungsgemäß.

### Beispiele

Folgende Abkürzungen werden im Folgenden verwendet:
- VE: voll entsalzt
- Q-: quaternisiert
- VP: N-Vinylpyrrolidon
- VI: N-Vinylimidazol
- VI*MeCl: Vinylimidazol mit Methylchlorid quaternisiert
- VI*DMS: Vinylimidazol mit Dimethylsulfat quaternisiert
- DMAEMA: Dimethylaminoethylmethacrylat
- TMAEMC: 2-Trimethylammoniumethylmethacrylat-Chlorid
- DADMAC: Diallyldimethylammoniumchlorid

### Beispiel 1

58.5 g VI wurden mit 465.79 g Catiofast^{®}CS (31 Gew.-%ige wässrige Lösung von Poly-DADMAC M_{w}= 140 00 g/mol) und 58.5 g VP gemischt. Die erhaltene Mischung wurde auf 90°C erhitzt und mit 150 Upm gerührt. Zuläufe 1 und 2 wurden in 2.5 Stunden zudosiert. Danach wurde die Reaktionsmischung auf 60° gekühlt, Zulauf 3 als Batch zugegeben und Zulauf 4 in 30 Minuten zudosiert. Diese Reaktionsmischung ließ man 1 Stunde bei 60°C nachreagieren. Dann wurden 5 g Phenonip^{®} zugegeben und die Mischung bis auf Raumtemperatur abkühlen gelassen.

### Zulauf 1:

356.2 g Wasser
3.27 g Wako^{®}V50

### Zulauf 2:

1.3 g Mercaptoethanol
46.80 g Wasser

### Zugabe 3:

2.39 g tert.-Butylhydroperoxid (70 Gew.-% ige Lösung)

### Zulauf 4:

1.83 g Natriumdisulfit
28.17 g VE-Wasser

Die Polymer-Kombinationen der Beispiele 2 bis 7 wurden analog Beispiel 1 hergestellt. Zur Herstellung der Kombination gemäß Beispiel 6 wurde Mercaptoethanol vorgelegt. Die jeweiligen Gewichtsverhältnisse der Monomere sind in untenstehender Tabelle angegeben.

### Beispiel 8

64.10 g Natriumcitrat wurden mit 500 g Catiofast^{®} CS (30 Gew.-%ige wässrige Poly-DADMAC-Lösung) gemischt. Diese Mischung wurde mit 0.89 g Schwefelsäure (50%ig) auf pH 6.8 eingestellt. Dann wurden 31 g von Zulauf 1 zugegeben und die Mischung bei 180 Upm 10 min eingerührt. Dann wurde 1 Stunde mit Stickstoff inertisiert und auf 65°C aufgeheizt. Nach Erreichen von 65°C wurden 4 g von Zulauf 2 zugegeben und 15 min polymerisiert. Danach wurde der restliche Zulauf 1 in 2 Stunden und gleichzeitig 11 g von Zulauf 2 in 3 Stunden zudosiert.

Danach ließ man die Mischung 4 Stunden bei 65° polymerisieren. Danach wurde die Reaktionsmischung auf 70°C aufgeheizt und nach Erreichen dieser Temperatur wurden die restlichen 15 g von Zulauf 2 innerhalb 30 min zudosiert. Danach ließ man noch 1,5 Stunden bei 70°C nachpolymerisieren. Danach wurde die Reaktionsmischung auf Raumtemperatur abgekühlt.

### Zulauf 1:

18.75g VP
37.5g 50 gew.-% ige Quat 311-Lösung (DMAEMA quaternisiert mit Diethylsulfat)
98.76g VE-Wasser

### Zulauf 2:

29.44g VE-Wasser
0.56g Wako^{®}V50

Die Polymer-Kombinationen der Beispiele 9 bis 12 wurden analog Beispiel 8 hergestellt. Beispiel 13 entspricht Beispiel 11 hinsichtlich des Monomerverhältnisses, allerdings wurde die Polymer-Kombination von Beispiel 13 in Batchfahrweise und mit einem geringeren Feststoffgehalt hergestellt.

| Bei spiel | Bestandteile | Gew. - Verhältnis Monomere | FG % | K-Wert | 0,2% WS leicht wachsiger Griff ++ 0,5% wachsiger Griff +++ | Shampoo-stabilität mit 0,5% WS/ WS 40°C | NaCl-Gehalt (Gew.-%) | Aussehen Shampoo | barkeit Hand Bei 0.2% WS in shampoo | Nasskämmbarkeit Meßwert Bei 0.2% WS in shampoo |
|---|---|---|---|---|---|---|---|---|---|---|
| 001 | VI/VP/Poly-DADMAC | 22,5:22,5:55 | 26,8 | 72,2 | ++ +++ | mind. 3 Monate stabil | 10% | milchig, leicht viskos-viskos, Struktur glatt | 1,2 | 63% |
| | | | | | | | | | | |
| 002 | VI/poly DADMAC | 40:60 | 741 | 74,2 | ++ | mind. 3 Monate mit 1 u. 1,5 Gew.-% NaCl stabil | 10% | milchig, leicht viskos, Struktur glatt | 1,5 | 56% |
| 003 | VI/VP/Poly-DADMAC | 25:25:50 | 26,7 | 72,2 | nein | mind. 3 Monate stabil | 1,0% | milchig, viskos, Struktur glatt | 1,3 | 54% |
| 004 | VI/ Poly DADMAC | 50:50 | 27,3 | 73 | ++ | mind. 3 Monate mit 1 u. 1,5 Gew.-% NaCl stabil | 1,0% | milchig, stark viskos, Struktur glatt | 1,5 | 52% |
| | | | | | | | | | | |
| 005 | VI/VP/Poly-DADMAC | 20:20:60 | 24,8 | 73,7 | ++ | mind. 3 Monate | stabil | milchig, viskos, Struktur glatt | 1,2 | 63% |
| 006 | VI/VP/Poly-DADMAC | 22,5:22,5:55 | 29,0 | 77 | 0,2% WS kaum wachsig 0,5%WS ++ | 3 Monate instabil | 1,0% | milchig, leicht viskos, Struktur glatt | 1,5 | 53% |
| 007 | wie 006 jedoch höherer FG | 22,5:22,5:55 | 36,3 | 78 | ++ | 3 Monate instabil und die telquel Probe auch instabil bei 50°C | 1,0% | milchig, leicht viskos, Struktur glatt | 1,2 | 74% |
| 008 | Poly-DADMAC/ VP /Quat 311/Na-citrat | 20/2,5/2,5/7,5 | 29,7 | | nein | mind. 3 Mon. stabil | 1,0% | milchig, viskos, Struktur glatt | 1,5 | 59% |
| 009 | Poly-DADMAC/ VP/ Quat 311/ Na-citrat | 20/5,0/5,0/7,5 | 37,7 | | nein | mind. 3 Mon. stabil | 1,0% | milchig, viskos-stark viskos, Struktur glatt | 1,5 | 53% |
| 010 | Poly-DADMAC/ Na-citrat | 17,5/10/10/6,5 VP/Quat 311/ | 43 | | nein | mind. 3 Mon. stabil | 1,0% | milchig, viskos, Struk tur glatt | 1,5 | 55% |
| 011 | Poly-DADMAC/ VP/TMAEMC/ Na-citrat | 20/5,0/5,0/7,5 | 40,5 | | ++ | mind. 3 Mon. stabil | 1,0% | milchig, viskos, Struk tur glatt | 1,3 | 60% |
| 012 | Poly-DADMAC/ VP/TMAEMC/ Na-citrat | 20/3,0/7,0/7,5 | 39 | | ++ | mind. 3 Mon. stabil | 1,0% | milchig, viskos, Struk tur glatt | 1,2 | 71% |
| 013 | Poly-DADMAC/ VP/TMAEMC/ Na-citrat Batchfahrweise | 20/5,0/5,0/7,5 | 35,8 | | ++ | mind. 3 Mon. stabil | 1,0% | milchig, viskos, Struk tur glatt | 1 | 82% |

| | | | | | | | | | | |
|---|---|---|---|---|---|---|---|---|---|---|
| FG: Feststoffgehalt WS: Anteil Polymer-Kombination, gerechnet als Feststoff | | | | | | | | | | |

### Beurteilung:

| Shampoo | Aussehen Shampoo | Naßkämmbarkeit behandelt von Hand (Note 1-3) | Kämmkraft-Abnahme | Griff der nassen Haare |
|---|---|---|---|---|
| 0.2 Gew.-% Beispiel 1 mit 1 Gew.-% NaCl | Stark trüb bis milchig, viskos, Struktur glatt | 1- | 66% ± 4 | Sehr gepflegt, leicht wachsig |
| 0.5 Gew.-% Beispiel 2 mit 1 Gew.-% NaCl | Milchig, leicht viskos-viskos, Struktur glatt | 1 | 80% ± 3 | Sehr gepflegt, wachsig |
| 1 Gew.-% Beispiel 3 mit 1 Gew.-% NaCl | Milchig, weiß, leicht viskos, Struktur glatt | 1 | 83% ± 2 | Sehr gepflegt, wachsig, etwas ölig |
| 0,2% WS Jaguar C 14 S mit 1 Gew.-% NaCl | Trüb, viskos, Struktur glatt | 2 | 25% ± 4 | gepflegt |
| 0,5% WS Jaguar C 14 S mit 1 Gew.-% NaCl | Trüb, viskos, Struktur glatt | 1-2 | 54 % ± 4 | Sehr gepflegt |
| 0,2% WS Jaguar C 14 S + 2% WS GE Silikon SM 2725 1 Gew.- % NaCl | Milchig, leicht viskos-viskos, Struktur glatt, n. 3 Wochen getrennt, da Viskosität zu niedrig | 1- | 68% ± 4 | Sehr gepflegt, wachsig, etwas ölig |
| 0,2% WS Jaguar C 13 S + 2% WS GE Silikon SM 2725 1,5 Gew.-% NaCl | Milchig, viskos, Struktur glatt | 1 | 74% ± 4 | sehr gepflegt, wachsig, etwas ölig |
| 0,2% WS Jaguar C 14 S + 1% WS GE Silikon SM 2725 1,5 Gew.-% NaCl | Milchig, viskos, Struktur glatt | 1 | 68% ± 4 | Sehr gepflegt, wachsig, etwas ölig |

### Bestimmung der K-Werte

Die K-Werte wurden nach Fikentscher, Cellulosechemie, Bd. 13, S. 58 bis 64 (1932) bei 25°C in wässriger Lösung gemessen und stellen ein Maß für das Molgewicht dar. Die Lösung der Polymere enthält 1g Polymer in 100 ml Lösung.

Die Messung des K-Wertes erfolgt in einer Micro-Ubbelohde-Kapillare Typ M Ic der Fa. Schott.

### Nasskämmbarkeit (europäische, gebleichte Haartressen):

### Bestimmung Blindwert

Vor der Bestimmung wurde die gebleichte Haartresse (Länge ca. 24 cm / Gewicht 2,7-3,3 g) zunächst zweimal mit Texapon^{®}NSO insgesamt 1 Minute shampooniert und 1 Minute ausgespült um eine definierte Nässe und Quellung der zu erreichen. Danach wurde die Tresse so vorgekämmt, bis keine Verhakungen der Haare mehr vorhanden waren.

Anschließend wurde die Tresse an der Halterung fixiert und mit der feinzinkigen Seite des Kammes in die feinzinkige Seite des Prüfkammes eingekämmt. Das Einlegen der Haare in den Prüfkamm erfolgte bei jeder Messung gleichmäßig und spannungsfrei. Die Messung wurde gestartet und mit Hilfe der Software EGRANUDO^{®} (Fa. Frank) ausgewertet. Die Messung wurde 5-10 mal wiederholt. Die Messungen wurden im Klimaraum bei ca. 65% relativer Feuchte und 21 °C durchgeführt.

Der errechnete Mittelwert wurde zusammen mit der Standardabweichung notiert.

### Shampoo-Rezeptur:

| | |
|---|---|
| 35,70g | Texapon^{®}NSO |
| 12,50g | Tego Betain L 7 |
| 0,5 g | Polymer-Kombination effektiv (0,5 g gerechnet als Feststoff) |
| 0,10g | Euxyl^{®}K 100 |
| ad 100g | Wasser |
| 1,00g | NaCl |

5 g des zu testenden Shampoos wurden aufgetragen, 1 Min. shampooniert, 1 Min. ausgespült, auf Filterpapier abgedrückt und gekämmt und der Messwert bestimmt.

### Auswertung:

Kämmkraftabnahme nass= 100-(Messwert*100/Blindwert); Angabe in %
Verwendete Geräte: Zug/ Druck-Prüfgerät der Fa. Frank

### Digitalwaage (Oberschalenwaage)

### Beispiele kosmetischer Zusammensetzungen:

### Haarkosmetische Zusammensetzung (allg.)

a) 0,01 bis 5 Gew.-% der Polymer-Kombination (gerechnet als Feststoff)
b) 25 bis 99,99 Gew.-% Wasser und/oder Alkohol
c) 0 bis 95,99 Gew.-% weitere Bestandteile

Unter Alkohol sind alle in der Kosmetik üblichen Alkohole zu verstehen, z.B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d.h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z.B. Konservierungsmittel, Parfümöle, Trübungsmittel, Wirkstoffe, UV-Filter, Pflegestoffe wie Panthenol, Collagen, Vitamine, Eiweiß-hydrolysate, Alpha- und Beta-Hydroxycarbonsäuren, Chitosan, Eiweißhydrolysate, Kosmetik-Polymere, Stabilisatoren, pH-Wert-Regulatoren, Farbstoffe, Viskositätsregulierer, Gelbildner, Farbstoffe, Salze, Feuchthaltemittel, Rückfetter, Komplexbildner und weitere übliche Additive.

### Shampooformulierung/Duschg/formulierung

Bevorzugte Shampooformulierungen oder Duschgelformulierungen enthalten
a) 0,01 bis 5 Gew.-% der Polymer-Kombination (gerechnet als Feststoff)
b) 25 bis 99,99 Gew.-% Wasser
c) 0 - 5 Gew.-% eines weiteren Konditioniermittels
d) 0 - 30 Gew.-% weitere kosmetische Bestandteile

In den Shampooformulierungen können zudem alle in Shampoos üblicherweise eingesetzten anionischen, neutralen, amphoteren oder kationischen Tenside verwendet werden mit den vorstehenden Maßgaben.

### Beispiel 1: Conditioner Shampoo mit PQ-10

| | |
|---|---|
| 35,70 g | Sodium Laureth Sulfate |
| 6,50 g | Cocamidopropyl Betaine |
| 0,20 g | Polymer-Kombination gemäß Beispiel 1 |
| 0,40 g | Polyquaternium-10 |
| 0,10 g | Konservierungsmittel |
| 0,10 g | Parfumöl / etherisches Öl |
| ad 100 g | Aqua dem. |

Gute Conditioner Shampoos werden auch erhalten, wenn anstelle der Polymer-Kombination gemäß Beispiel 1 die Polymer-Kombinationen der Beispiele 2 bis 13 verwendet werden.

### Beispiel 2: Conditioner Shampoo mit GHTC

| | |
|---|---|
| 35,70 g | Sodium Laureth Sulfate |
| 6,50 g | Cocamidopropyl Betaine |
| 0,50 g | Polymer-Kombination gemäß Beispiel 1 |
| 0,20 g | Guarhydroxypropyltrimonium Chloride |
| 0,10 g | Konservierungsmittel |
| 0,10 g | Parfumöl / etherisches Öl |
| ad 100 g | Aqua dem. |

Gute Conditioner Shampoos werden auch erhalten, wenn anstelle der Polymer-Kombination gemäß Beispiel 1 die Polymer-Kombinationen der Beispiele 2 bis 13 verwendet werden.

### Beispiel 3: Conditioner Shampoo mit Polyquaternium

| | |
|---|---|
| 35,70 g | Sodium Laureth Sulfate |
| 6,50 g | Cocamidopropyl Betaine |
| 0,20 g | Polymer-Kombination gemäß Beispiel 1 |
| 0,30 g | Polyquaternium-44 oder PQ-67 |
| 0,10 g | Konservierungsmittel |
| 0,10 g | Parfumöl / etherisches Öl |
| ad 100 g | Aqua dem. |

Gute Conditioner Shampoos werden auch erhalten, wenn anstelle der Polymer-Kombination gemäß Beispiel 1 die Polymer-Kombinationen der Beispiele 2 bis 13 verwendet werden.

### Beispiel 4: Shampoo

### Phase A

| | |
|---|---|
| 15,00 g | Cocamidopropyl Betaine |
| 10,00 | g Disodium Cocoamphodiacetate |
| 5,00 g | Polysorbate 20 |
| 5,00 g | Decyl Glucoside |
| 0,20 g | Polymer-Kombination gemäß Beispiel 1 |
| 0,10 g | Parfumöl / etherisches Öl |
| q.s. | Konservierungsmittel |
| 2,00 g | Laureth-3 |
| add 100 | Aqua dem. |
| q.s. | Citric Acid |

### Phase B

| | |
|---|---|
| 3,00 g | PEG-150 Distearate |

### Herstellung

Komponenten der Phase A einwiegen und lösen; pH-Wert auf 6-7 einstellen. Phase B zugeben und auf 50°C erwärmen. Auf Raumtemperatur abkühlen lassen unter Rühren.

Gute Shampoos werden auch erhalten, wenn anstelle der Polymer-Kombination gemäß Beispiel 1 die Polymer-Kombinationen der Beispiele 2 bis 13 verwendet werden.

### Beispiel 5: Shampoo

| | |
|---|---|
| 30,00 g | Sodium Laureth Sulfate |
| 6,00 g | Sodium Cocoamphoacetate |
| 0,50 g | Polymer-Kombination gemäß Beispiel 1 |
| 3,00 g | Sodium Laureth Sulfate, Glycol Distearate, Cocamide MEA, Laureth-10 |
| 2,00 g | Dimethicone |
| q.s. | Parfum |
| q.s. | Konservierungsmittel |
| q.s. | Citric Acid |
| 1,00 g | Sodium Chloride |
| add 100 | Aqua dem. |

Gute Shampoos werden auch erhalten, wenn anstelle der Polymer-Kombination gemäß Beispiel 1 die Polymer-Kombinationen der Beispiele 2 bis 13 verwendet werden.

### Beispiel 6: Duschgel

| | |
|---|---|
| 20,00 g | Ammonium Laureth Sulfate |
| 15,00 g | Ammonium Lauryl Sulfate |
| 0,50 g | Polymer-Kombination gemäß Beispiel 1 |
| 0,50 g | Polyquaternium-7 |
| 2,50 g | Sodium Laureth Sulfate, Glycol Distearate, Cocamide MEA, Laureth-10 |
| 0,10 g | Parfumöl / etherisches Öl |
| q.s. | Konservierungsmittel |
| 0,50 g | Sodium Chloride |
| add 100 | Aqua dem. |

Gute Duschgele werden auch erhalten, wenn anstelle der Polymer-Kombination gemäß Beispiel 1 die Polymer-Kombinationen der Beispiele 2 bis 13 verwendet werden.

### Beispiel 7: Duschgel

| | |
|---|---|
| 40,00 g | Sodium Laureth Sulfate |
| 5,00 g | Decyl Glucoside |
| 5,00 g | Polymer-Kombination gemäß Beispiel 1 |
| 1,00 g | Panthenol |
| 0,10 g | Parfumöl / etherisches Öl |
| q.s. | Konservierungsmittel |
| q.s. | Citric Acid |
| 2,00 g | Sodium Chloride |
| add 100 | Aqua dem. |

Gute Duschgele werden auch erhalten, wenn anstelle der Polymer-Kombination gemäß Beispiel 1 die Polymer-Kombinationen der Beispiele 2 bis 13 verwendet werden.

### Beispiel 8: Shampoo

| | |
|---|---|
| 12,00 g | Sodium Laureth Sulfate |
| 1,50 g | Decyl Clucoside |
| 0,50 g | Polymer-Kombination gemäß Beispiel 1 |
| 5,00 g | Coco-Glucoside Glyceryl Oleate |
| 2,00 g | Sodium Laureth Sulfate, Glycol Distearate, Cocomide MEA, Laureth-10 |
| q.s. | Konservierungsmittel |
| q.s. | Sunset Yellow C. I. 15 985 |
| 0,10 g | Parfumöl / etherisches Öl |
| 1,00 g | Sodium Chloride |
| add 100 | Aqua dem. |

Gute Shampoos werden auch erhalten, wenn anstelle der Polymer-Kombination gemäß Beispiel 1 die Polymer-Kombinationen der Beispiele 2 bis 13 verwendet werden.

Die für die erfindungsgemäße Verwendung geeigneten Polymerkombinationen eignen sich auch für die Verwendung in Haarstyling-Zubereitungen, insbesondere Haarschäume (Aerosolschäume mit Treibgas und Pumpschäüme ohne Treibgas), Haarsprays (Pumpssprays ohne Treibgas) und Haargele.

Treibmittel sind die üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

### Aerosolhaarschaum

a) 0,1 bis 10 Gew.-% eines Kosmetik-Polymers
b) 55 bis 99,8 Gew.-% Wasser und/oder Alkohol
c) 5 bis 20 Gew.-% eines Treibmittels
d) 0,1 bis 5 Gew.-% einer erfindungsgemäß geeigneten Polymerkombination
e) 0 bis 10 Gew.-% weitere Bestandteile

Als weitere Bestandteile können unter anderem alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCl-Nomenklatur) sind Laurethe, z.B. Laureth-4; Cetethe, z.B. Cetheth-1, Polyethylenglycolcetylether; Cetearethe, z.B. Cetheareth-25, Polyglycolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammoniumdihydrogenphosphat, Cetyltrimoniumchlorid, Cetyltrimmoniumbromid, Cocotrimoniummethylsulfat, Quaternium-1 bis x (INCl).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:

### Beispiel 9: Aerosolhaarschaum

| | |
|---|---|
| 2.00 g | Cocotrimonium Methosulfate |
| 0,10 g | Parfumöl / etherisches Öl |
| 3,50 g | Festigerpolymer z.B. Polyquaternium-46, PQ-44, VP/Methacrylamide/ Vinyl Imidazole Copolymer, etc. |
| 0,80 g | Polymer-Kombination gemäß Beispiel 1 |
| q.s. | Preservative |
| 75,00 g | Wasser dem. |
| 10,00 g | Propane/Butane (3.5 bar) |

Gute Aerosolhaarschäume werden auch erhalten, wenn anstelle der Polymer-Kombination gemäß Beispiel 1 die Polymer-Kombinationen der Beispiele 2 bis 13 verwendet werden.

### Stylinggel

a) 0,1 bis 10 Gew.-% eines Kosmetik-Polymers
b) 60 bis 99,85 Gew.-% Wasser und/oder Alkohol
c) 0,05 bis 10 Gew.-% eins Gelbildners
d) 0,1 bis 5 Gew.-% einer erfindungsgemäß geeigneten Polymerkombination
e) 0 bis 20 Gew.-% weitere Bestandteile

Als Gelbildner können alle in der Kosmetik üblichen Gelbildner eingesetzt werden. Hierzu zählen leicht vernetzte Polyacrylsäure, beispielsweise Carbomer (INCl), Cellulosederivate, z.B. Hydroxypropylcellulose, Hydroxyethylcellulose, kationisch modifizierte Cellulosen, Polysaccharide, z.B. Xanthum Gummi, Caprylic/Capric Triglyceride, Sodium acrylates Copolymer, Polyquaternium-32 (and) Paraffinum Liquidum (INCl), Sodium Acrylates Copolymer (and) Paraffinum Liquidum (and) PPG-1 Trideceth-6, Acrylamidopropyl Trimonium Chloride/Acrylamide Copolymer, Steareth-10 Allyl Ether Acrylates Copolymer, Polyquaternium-37 (and) Paraffinum Liquidum (and) PPG-1 Trideceth-6, Polyquaternium-37 (and) Propylene Glycole Dicaprate Dicaprylate (and) PPG-1 Trideceth-6, Polyquaternium-7, Polyquaternium-44, Polyquaternium-67.

Gute Stylinggele werden auch erhalten, wenn anstelle der Polymer-Kombination gemäß Beispiel 1 die Polymer-Kombinationen der Beispiele 2 bis 13 verwendet werden.

### Beispiel 10: Haarstylinggel

### Phase A

| | |
|---|---|
| 0,50 g | Carbomer oder Acrylates/C10-30 Alkyl Acrylate Crosspolymer |
| 86,40 g | Wasser dem. |

### Phase B

| | |
|---|---|
| 0,70 g | Triethanolamine |

### Phase C

| | |
|---|---|
| 6,00 g | Festigerpolymer z.B. VP/MethacrylamideNinyl Imidazole Copolymer |
| 5,00g | PVP |
| 0,20 g | PEG-25 PABA |
| 0,50 g | Polymer-Kombination gemäß Beispiel 1 |
| 0,10 g | Parfumöl / etherisches Öl |
| q.s. | PEG-14 Dimethicone |
| q.s. | Konservierungsmittel |
| 0,10 g | Tocopheryl Acetate |

Gute Stylinggele werden auch erhalten, wenn anstelle der Polymer-Kombination gemäß Beispiel 1 die Polymer-Kombinationen der Beispiele 2 bis 13 verwendet werden.

### Beispiel 11: Haarstylinggel

### Phase A

| | |
|---|---|
| 0,50 g | Carbomer oder Acrylates/C10-30 Alkyl Acrylate Crosspolymer |
| 91,20 g | Wasser dem. |

### Phase B

| | |
|---|---|
| 0,90 g | Tetrahydroxypropyl Ethylenediamine |

### Phase C

| | |
|---|---|
| 7,00 g | VPNA Copolymer |
| 0,40 g | Polymer-Kombination gemäß Beispiel 1 |
| 0,20 g | Parfumöl / etherisches Öl |
| q.s. | Konservierungsmittel |
| 0,10 g | Propylene Glycol |

Gute Stylinggele werden auch erhalten, wenn anstelle der Polymer-Kombination gemäß Beispiel 1 die Polymer-Kombinationen der Beispiele 2 bis 13 verwendet werden.

### Beispiel 12: Hair Wax Cream

| | |
|---|---|
| 6,00 g | Caprylic/Capric Triglyceride |
| 3,00 g | Glyceryl Stearate |
| 2,00 g | Cetyl Alcohol |
| 3,50 g | Polymer-Kombination gemäß Beispiel 1 |
| 0,50 g | Cremophoer A6 |
| 0,70 g | Cremophor A25 |
| 0,50 g | Dimethicone |
| 0,50 g | Vitamin E Acetate |
| 2,00 g | Caprylic/Capric Triglyceride and Sodiumacrylates Copolymer |
| 1,00 g | D-Panthenol USP |
| 0,10 g | EDTA |
| 10,00 g | Festigerpolymer |
| q.s. | Konservierungsmittel |
| ad 100 g | Wasser dem. |

Gute Hair Wax Creams werden auch erhalten, wenn anstelle der Polymer-Kombination gemäß Beispiel 1 die Polymer-Kombinationen der Beispiele 2 bis 13 verwendet werden.

### Beispiel 13: Haarpudding

| | |
|---|---|
| 3,00 g | Kollicoat IR (BASF) |
| q.s. | Konservierungsmittel |
| 2,00 g | Festigerpolymer |
| 4,00 g | Acrylates/beheneth-25 Methacrylate Copolymer |
| 0,70 g | Polymer-Kombination gemäß Beispiel 1 |
| 0,50 g | Dimethicone Copolyol |
| 0,10 g | EDTA |
| 0,20 g | Benzophenone-4 |
| ad 100 g | Wasser dem. |

Gute Haarpuddings werden auch erhalten, wenn anstelle der Polymer-Kombination gemäß Beispiel 1 die Polymer-Kombinationen der Beispiele 2 bis 13 verwendet werden.

### Beispiel 14: Sprühgel

### Phase A

| | |
|---|---|
| 1,25 g | Festigerpolymer |
| 96,15 g | Aqua dem. |

### Phase B

| | |
|---|---|
| 0,70 g | Acrylates/Steareth-20 Itaconate Copolymer |
| 0,10 g | Propylene Glycol |
| 0,50 g | Polymer-Kombination gemäß Beispiel 1 |
| 0,10 g | Glycerin |
| 0,10 g | Parfümöl / etherisches Öl |
| q.s. | Konservierungsmittel |

### Phase C

| | |
|---|---|
| 0,70 g | Triethanolamine |

Gute Sprühgele werden auch erhalten, wenn anstelle der Polymer-Kombination gemäß Beispiel 1 die Polymer-Kombinationen der Beispiele 2 bis 13 verwendet werden.

Eine erfindungsgemäß für Styling-Sprays geeignete Zubereitung kann beispielsweise wie folgt zusammengesetzt sein:

### Beispiel 15: Pumphaarspray

| | |
|---|---|
| 11,20 g | PEG/PPG-25/25 Dimethicone/Acrylates Copolymer |
| 2,80 g | VPNA Copolymer |
| 1,34 | g Aminomethyl Propanol |
| 0,30 g | Polymer-Kombination gemäß Beispiel 1 |
| 0,10 g | Parfumöl / etherisches Öl |
| 11,26 g | Aqua dem. |
| 73,00 g | Alcohol |

Gute Pumphaarsprays werden auch erhalten, wenn anstelle der Polymer-Kombination gemäß Beispiel 1 die Polymer-Kombinationen der Beispiele 2 bis 13 verwendet werden.

### Beispiel 16: Pumphaarspray VOC55

| | |
|---|---|
| 2,00 g | VP/Methacrylamide/ Vinyl Imidazole Copolymer |
| 1,90 g | Polyquaternium-46 |
| 2,00 g | Polymer-Kombination gemäß Beispiel 1 |
| 0,10 g | Parfumöl / etherisches Öl |
| 55,00 g | Alcohol |
| 39,00 g | Aqua dem. |

Gute Pumphaarsprays VOC 55 werden auch erhalten, wenn anstelle der Polymer-Kombination gemäß Beispiel 1 die Polymer-Kombinationen der Beispiele 2 bis 13 verwendet werden.

### Hautkosmetische Zusammensetzungen

### Beispiel 17: Flüssiges Makeup

### Phase A

| | |
|---|---|
| 1,70 g | Glyceryl Stearate |
| 1,70 g | Cetyl Alcohol |
| 1,70 g | Ceteareth-6 |
| 1,70 g | Ceteareth-25 |
| 5,20 g | Caprylic/Capric Triglyceride |
| 5,20 g | Mineral Oil oder Luvitol^{®} Lite (INCl Hydrogenated Polyisobutene) |

### Phase B

| | |
|---|---|
| q.s. | Konservierungsmittel |
| 4,30 g | Propylene Glycol |
| 2,50 g | Polymer-Kombination gemäß Beispiel 1 |
| 59,50 g | Aqua dem. |

### Phase C

| | |
|---|---|
| 0,10 g | Parfumöl / etherisches Öl |

### Phase D

| | |
|---|---|
| 2,00 g | Iron Oxides |
| 12,00 g | Titanium Dioxide |

Gute Flüssig-Make-ups werden auch erhalten, wenn anstelle der Polymer-Kombination gemäß Beispiel 1 die Polymer-Kombinationen der Beispiele 2 bis 13 verwendet werden.

### Beispiel 18: Eyeliner

### Phase A

| | |
|---|---|
| 40,60 g | dest. Wasser |
| 0,20 | g Disodium EDTA |
| q.s. | Konservierungsmittel |

### Phase B

| | |
|---|---|
| 0,60 g | Xanthan Gum |
| 0,40 g | Veegum |
| 3,00 g | Butylene Glycol |
| 0,20 g | Polysorbate-20 |

### Phase C

| | |
|---|---|
| 15,00 g | Iron oxide / Al Powder / Silica (z.B. Sicopearl^{®} Fantastico Gold von BASF) |

### Phase D

| | |
|---|---|
| 10,00 g | Aqua dem. |
| 25,00 g | Festigerpolymer (z.B. Polyurethane-1 oder VP/Methacrylamide/ Vinyl Imidazole Copolymer, etc.) |
| 5,00 g | Polymer-Kombination gemäß Beispiel 1 |

Gute Eyeliner werden auch erhalten, wenn anstelle der Polymer-Kombination gemäß Beispiel 1 die Polymer-Kombinationen der Beispiele 2 bis 13 verwendet werden.

### Beispiel 19: Sonnenschutz-Gel

### Phase A

| | |
|---|---|
| 0,90 g | Polymer-Kombination gemäß Beispiel 1 |
| 8,00 g | Octyl Methoxycinnamate |
| 5,00 g | Octocrylene |
| 0,80 g | Octyl Triazone |
| 2,00 g | Butyl Methoxydibenzoylmethane |
| 2,00 g | Tocopheryl Acetate |
| 0,10 g | Parfumöl / etherisches Öl |

### Phase B

| | |
|---|---|
| 0,30 g | Acrylates/C10-30 Alkyl Acrylate Crosspolymer |
| 0,20 g | Carbomer |
| 5,00 g | Glycerin |
| 0,20 | g Disodium EDTA |
| q.s. | Konservierungsmittel |
| 75,30 g | Aqua dem. |

### Phase C

| | |
|---|---|
| 0,20 g | Sodium Hydroxide |

Gute Sonnenschutzgele werden auch erhalten, wenn anstelle der Polymer-Kombination gemäß Beispiel 1 die Polymer-Kombinationen der Beispiele 2 bis 13 verwendet werden.

### Beispiel 20: Sonnenschutzemulsion mit TiO₂ und ZnO₂

### Phase A

| | |
|---|---|
| 1,00 g | PEG-7 Hydrogenated Castor Oil |
| 5,00 g | Polymer-Kombination gemäß Beispiel 1 |
| 2,00 g | PEG-45/Dodecyl Glycol Copolymer |
| 3,00 g | Isopropyl Myristate |
| 7,90 g | Jojoba (Buxus Chinensis) Oil |
| 4,00 g | Octyl Methoxycinnamate |
| 2,00 g | 4-Methylbenzylidene Camphor |
| 3,00 g | Titanium Dioxide, Dimethicone |
| 1,00 g | Dimethicone |
| 5,00 g | Zinc Oxide, Dimethicone |

### Phase B

| | |
|---|---|
| 0,20 | g Disodium EDTA |
| 5,00 g | Glycerin |
| q.s. | Konservierungsmittel |
| 60,80 g | Aqua dem. |

### Phase C

| | |
|---|---|
| 0,10 g | Parfümöl / etherisches Öl |

Gute Sonnenschutzemulsionen werden auch erhalten, wenn anstelle der Polymer-Kombination gemäß Beispiel 1 die Polymer-Kombinationen der Beispiele 2 bis 13 verwendet werden.

### Beispiel 21: Gesichtswasser

### Phase A

| | |
|---|---|
| 3,00 g | Polymer-Kombination gemäß Beispiel 1 |
| 0,10 g | Parfümöl / etherisches Öl |
| 0,30 g | Bisabolol |

### Phase B

| | |
|---|---|
| 3,00 g | Glycerin |
| 1,00 g | Hydroxyethyl Cetyldimonium Phosphate |
| 5,00 g | Whitch Hazel (Hamamelis Virginiana) Distillate |
| 0,50 g | Panthenol |
| q.s. | Konservierungsmittel |
| 87,60 g | Aqua dem. |

Gute Gesichtswässer werden auch erhalten, wenn anstelle der Polymer-Kombination gemäß Beispiel 1 die Polymer-Kombinationen der Beispiele 2 bis 13 verwendet werden.

### Beispiel 22: Gesichtswaschpaste mit Peelingeffekt

### Phase A

| | |
|---|---|
| 73,00 g | Aqua dem. |
| 1,50 g | Carbomer |
| q.s. | Konservierungsmittel |

### Phase B

| | |
|---|---|
| q.s. | Parfümöl |
| 7,00 g | Potassium Cocoyl Hydrolyzed Protein |
| 4,00 g | Polymer-Kombination gemäß Beispiel 1 |

### Phase C

| | |
|---|---|
| 1,50 g | Triethanolamine |

### Phase D

| | |
|---|---|
| 13,00 g | Polyethylene (Luwax A™ von BASF) |

Gute Gesichtswaschpasten werden auch erhalten, wenn anstelle der Polymer-Kombination gemäß Beispiel 1 die Polymer-Kombinationen der Beispiele 2 bis 13 verwendet werden.

### Beispiel 23: Seife

### Phase A

| | |
|---|---|
| 25,00 g | Potassium Cocoate |
| 20,00 | g Disodium Cocoamphodiacetate |
| 2,00 g | Lauramide DEA |
| 1,0 g | Glycol Stearate |
| 2,00 g | Polymer-Kombination gemäß Beispiel 1 |
| 50,00 g | Aqua dem. |
| q.s. | Citric Acid |

### Phase B

| | |
|---|---|
| q.s. | Konservierungsmittel |
| 0,10 g | Parfumöl / etherisches Öl |

Gute Seifen werden auch erhalten, wenn anstelle der Polymer-Kombination gemäß Beispiel 1 die Polymer-Kombinationen der Beispiele 2 bis 13 verwendet werden.

### Beispiel 24: Gesichtsreinigungsmilch Typ O/W

### Phase A

| | |
|---|---|
| 1,50 g | Ceteareth-6 |
| 1,50 g | Ceteareth-25 |
| 2,00 g | Glyceryl Stearate |
| 2,00 g | Cetyl Alcohol |
| 10,00 g | Mineral Oil |

### Phase B

| | |
|---|---|
| 5,00 g | Propylene Glycol |
| q.s. | Konservierungsmittel |
| 1,00 g | Polymer-Kombination gemäß Beispiel 1 |
| 66,30 g | Aqua dem. |

### Phase C

| | |
|---|---|
| 0,20 g | Carbomer |
| 10,00 g | Cetearyl Octanoate |

### Phase D

| | |
|---|---|
| 0,40 g | Tetrahydroxypropyl Ethylenediamine |

### Phase E

| | |
|---|---|
| 0,10 g | Parfumöl / etherisches Öl |
| 0,10 g | Bisabolol |

Gute Gesichtsreinigungsmilche werden auch erhalten, wenn anstelle der Polymer-Kombination gemäß Beispiel 1 die Polymer-Kombinationen der Beispiele 2 bis 13 verwendet werden.

### Beispiel 25: Transparente Seife

| | |
|---|---|
| 4,20 g | Sodium Hydroxide |
| 3,60 g | dest. Wasser |
| 10,00 g | Polymer-Kombination gemäß Beispiel 1 |
| 22,60 g | Propylene Glycol |
| 18,70 g | Glycerin |
| 5,20 g | Cocoamide DEA |
| 2,40 g | Cocamine Oxide |
| 4,20 g | Sodium Lauryl Sulfate |
| 7,30 g | Myristic Acid |
| 16,60 g | Stearic Acid |
| 5,20 g | Tocopherol |

Gute transparente Seifen werden auch erhalten, wenn anstelle der Polymer-Kombination gemäß Beispiel 1 die Polymer-Kombinationen der Beispiele 2 bis 13 verwendet werden.

### Beispiel 26: Rasierschaum

| | |
|---|---|
| 6,00 g | Ceteareth-25 |
| 5,00 g | Poloxamer 407 |
| 52,00 g | Aqua dem. |
| 1,00 g | Triethanolamine |
| 5,00 g | Propylene Glycol |
| 1,00 g | PEG-75 Lanolin Oil |
| 5,00 g | Polymer-Kombination gemäß Beispiel 1 |
| q.s. | Konservierungsmittel |
| 0,10 g | Parfumöl / etherisches Öl |
| 25,00 g | Sodium Laureth Sulfate |

### Abfüllung: 90 Teile Wirksubstanz und 10 Teile Propan/Butan-Mischung 25:75.

Gute Rasierschäume werden auch erhalten, wenn anstelle der Polymer-Kombination gemäß Beispiel 1 die Polymer-Kombinationen der Beispiele 2 bis 13 verwendet werden.

### Beispiel 27: After Shave Balsam

### Phase A

| | |
|---|---|
| 0,25 g | Acrylates/C10-30 Alkyl Acrylate Crosspolymer |
| 1,50 g | Tocopheryl Acetate |
| 0,20 g | Bisabolol |
| 10,00 g | Caprylic/Capric Triglyceride |
| q.s. | Parfum |
| 1,00 g | Polymer-Kombination gemäß Beispiel 1 |

### Phase B

| | |
|---|---|
| 1,00 g | Panthenol |
| 15,00 g | Alcohol |
| 5,00 g | Glycerin |
| 0,05 g | Hydroxyethyl Cellulose |
| 1,90 g | Polymer-Kombination gemäß Beispiel 1 |
| 64,02 g | dest. Wasser |

### Phase C

| | |
|---|---|
| 0,08 g | Sodium Hydroxide |

Gute After-Shave-Balsams werden auch erhalten, wenn anstelle der Polymer-Kombination gemäß Beispiel 1 die Polymer-Kombinationen der Beispiele 2 bis 13 verwendet werden.

### Beispiel 28: Pflegecreme

### Phase A

| | |
|---|---|
| 2,00 g | Ceteareth-6 |
| 2,00 g | Ceteareth-25 |
| 2,00 g | Cetearyl Alcohol |
| 3,00 g | Glyceryl Stearate SE |
| 5,00 g | Mineral Oil |
| 4,00 g | Jojoba (Buxus Chinensis) Oil |
| 3,00 g | Cetearyl Octanoate |
| 1,00 g | Dimethicone |
| 3,00 g | Mineral Oil, Lanolin Alcohol |

### Phase B

| | |
|---|---|
| 5,00 g | Propylene Glycol |
| 0,50 g | Veegum |
| 1,00 g | Panthenol |
| 1,70 g | Polymer-Kombination gemäß Beispiel 1 |
| 6,00 g | Polyquaternium-44 |
| q.s. | Konservierungsmittel |
| 60,80 g | Auqa dem. |

### Phase C

| | |
|---|---|
| q.s. | Parfum |

Gute Pflegecremes werden auch erhalten, wenn anstelle der Polymer-Kombination gemäß Beispiel 1 die Polymer-Kombinationen der Beispiele 2 bis 13 verwendet werden.

### Mund- und Zahnpflegezubereitungen

### Beispiel 29: Zahnpaste

### Phase A

| | |
|---|---|
| 34,79 g | Aqua dem. |
| 3,00 g | Polymer-Kombination gemäß Beispiel 1 |
| 20,00 g | Glycerin |
| 0,76 g | Sodium Monofluorophosphate |

### Phase B

| | |
|---|---|
| 1,20 g | Sodium Carboxymethylcellulose |

### Phase C

| | |
|---|---|
| 0,80 g | Aromaöl |
| 0,06 g | Saccharin |
| q.s. | Konservierungsmittel |
| 0,05 g | Bisabolol |
| 1,00 g | Panthenol |
| 0,50 g | Tocopheryl Acetate |
| 2,80 g | Silica |
| 1,00 g | Sodium Lauryl Sulfate |
| 7,90 g | Dicalciumphosphate Anhydrate |
| 25,29 g | Dicalciumphosphate Dihydrate |
| 0,45 g | Titanium Dioxide |

Gute Zahnpasten werden auch erhalten, wenn anstelle der Polymer-Kombination gemäß Beispiel 1 die Polymer-Kombinationen der Beispiele 2 bis 13 verwendet werden.

### Beispiel 30: Mundwasser

### Phase A

| | |
|---|---|
| 2,00 g | Aromaöl |
| 4,50 g | Polymer-Kombination gemäß Beispiel 1 |
| 1,00 g | Bisabolol |
| 30,00 g | Alcohol |

### Phase B

| | |
|---|---|
| 0,20 g | Saccharin |
| 5,00 g | Glycerin |
| q.s. | Konservierungsmittel |
| 5,00 g | Poloxamer 407 |
| 52,30 g | Aqua dem. |

Gute Mundwässer werden auch erhalten, wenn anstelle der Polymer-Kombination gemäß Beispiel 1 die Polymer-Kombinationen der Beispiele 2 bis 13 verwendet werden.

### Beispiel 37: Prothesenhaftmittel

### Phase A

| | |
|---|---|
| 0,20 g | Bisabolol |
| 1,00 g | Beta-Carotene |
| q.s. | Aromaöl |
| 20,00 g | Cetearyl Octanoate |
| 5,00 g | Silica |
| 33,80 g | Mineral Oil |

### Phase B

| | |
|---|---|
| 5,00 g | Polymer-Kombination gemäß Beispiel 1 |
| 35,00 g | PVP (20%ige Lösung in Wasser) |

Gute Prothesenhaftmittel werden auch erhalten, wenn anstelle der Polymer-Kombination gemäß Beispiel 1 die Polymer-Kombinationen der Beispiele 2 bis 13 verwendet werden.

Die folgenden Beispiele werden beispielsweise hergestellt, wie in US 6,451,300, Spalten 31 und 32 beschrieben.

| Beispiel 50 | Gew.-% |
|---|---|
| | |
| Ammoniumlaurethsulfat | 12 |
| Ammoniumlaurylsulfat | 8 |
| Polymer-Kombination gemäß Beispiel 1 | 0,4 |
| PEG-90M³ (INCl) | 0,5 |
| Zinkpyrithion⁴ (optional) | 1 |
| 1-Decen-Hompolymer⁵ | 0,3 |
| Trimethylpropantricaprylat/caprat⁶ | 0,1 |
| Dimethicone⁷ (optional) | 2,0 |
| Ethylenglykoldistearat | 2,0 |
| Kokosamid MEA | 0,8 |
| Cetylalkohol | 0,9 |
| Wasser und Mindermengen | q.s. |

| | |
|---|---|
| ³ PEG M_{w} ca. 4*10⁶ g/mol ⁴ Zinkpyrithion mit mittlerer Teilchengröße von ca. 2,5 µm; ⁵ Puresyn 6 (Hydrogenated Polydecene) ⁶ Mobil^{®}P43 ⁷ Visasil^{®}330.000 cSt (General Electric Silicones). | |

Gute Shampoos werden auch erhalten, wenn anstelle der Polymer-Kombination gemäß Beispiel 1 die Polymer-Kombinationen der Beispiele 2 bis 13 verwendet werden.

| Beispiel | 63 | 64 |
|---|---|---|
| | Gew.-% | Gew.-% |
| Ammoniumlaurethsulfat | 12 | 10 |
| Ammoniumlaurylsulfat | 6 | 6 |
| Polyquaternium-10 | - | 0,25 |
| Polymer-Kombination gemäß Beispiel 1 | 0,25 | 0,25 |
| PEG-7M⁴ (INCl) | - | 0,1 |
| PEG-90M⁵(INCl) | 0,1 | - |
| Zinkpyrithion⁶ (optional) | 1 | 1 |
| 1 -Decen-Hompolymer⁷ | 0,4 | 0,4 |
| Trimethylpropantricaprylat/caprat⁸ | - | 0,1 |
| Dimethicone⁹ (optional) | 1,15 | 1,35 |
| Ethylenglykoldistearat | 1,0 | 1,5 |
| Kokosamid MEA | 1,1 | 0,8 |
| Cetylalkohol | 0,6 | 0,9 |
| Wasser und Mindermengen | q.s. | q.s. |

| | | |
|---|---|---|
| ⁴ Polyox^{®}WSR N-750 ⁵ Polyox^{®}WSR N-301 ⁶ Zinkpyrithion mit mittlerer Teilchengröße von ca. 2,5 µm; ⁷ Puresyn 6 (Hydrogenated Polydecene) ⁸ Mobil^{®}P43 ⁹ Visasil^{®}330.000 cSt (General Electric Silicones) | | |

Gute Shampoos werden jeweils auch erhalten, wenn anstelle der Polymer-Kombination gemäß Beispiel 1 die Polymer-Kombinationen der Beispiele 2 bis 13 verwendet werden.

| Beispiel | 90 |
|---|---|
| | Gew.-% |
| Natriumlaurethsulfat | 10 |
| Natriumlaurylsulfat | 6 |
| Ethylenglykoldistearat | 1,5 |
| Kokosamid MEA | 0,8 |
| Cetylalkohol | 0,9 |
| Polymer-Kombination gemäß Beispiel 1 | 0,5 |
| Dimethicone⁹ (optional) | 2,35 |
| Trimethylpropantricaprylat/caprat⁸ | 0,1 |
| 1 -Decen-Hompolymer⁷ | 0,4 |
| Zinkpyrithion¹⁰ (optional) | 1,0 |
| Natriumcitrat | 0,2 |
| Zitronensäure | 0,22 |
| Natriumchlorid | 1,475 |
| Parfum | 0,7 |
| Natriumbenzoat | 0,25 |
| Kathon^{®}CG | 0,0005 |
| Wasser | q.s. |

| | |
|---|---|
| ¹⁰ Zinkpyrithion mit mittlerer Teilchengröße von ca. 2,5 µm; ⁷ Puresyn 6 (Hydrogenated Polydecene) ⁸ Mobil^{®}P43 ⁹ Visasil^{®}330.000 cSt (General Electric Silicones) | |

Gute Shampoos werden auch erhalten, wenn anstelle der Polymer-Kombination gemäß Beispiel 1 die Polymer-Kombinationen der Beispiele 2 bis 13 verwendet werden.

### Beispiel 103 Hair Repair Shampoo "Smooth & Silky"

| Phase | Gew.-% | Inhaltsstoff | Bezugsquelle | INCl Bezeichnung |
|---|---|---|---|---|
| A | 0,5 | Polymer-Kombination gemäß Beispiel 1 | | |
| | 47,3 | Wasser voll entsalzt | | Aqua |
| B | 12,5 | Tego^{®}Betain L7 | Evonik Goldschmidt GmbH | Cocamidopropyl Betaine |
| | 35,7 | Texapon^{®}NSO | Cognis Deutschland GmbH & Co. KG | Sodium Laureth Sulfate |
| | 0,10 | Euxyl^{®}K 100 | Schülke & Mayr GmbH | Benzyl Alcohol (and) Methyl chloroisothiazolinone (and) Methyl chloroisothiazolinone |
| | 0,30 | Parfum | | Fragrance |
| | 0,50 | D-Panthenol USP | BASF SE | Panthenol |
| | 0,10 | Edeta®BD | BASF SE | Disodium EDTA |
| | 1,5 | NaCl | | Sodium Chloride |

Herstellung: zur gemischten Phase A die Komponenten der Phase B nacheinander zugeben und so lange rühren, bis alle komponenten vollständig gelöst sind. Eigenschaften: pH-Wert 6.5, Viskosität: 9,000 mPa·s (Brookfield DV II+ sp. 4/20 Upm)

Gute Shampoos werden auch erhalten, wenn anstelle der Polymer-Kombination gemäß Beispiel 1 die Polymer-Kombinationen der Beispiele 2 bis 13 verwendet werden.

## Patentansprüche

1. Zusammensetzung enthaltend
a) wenigstens ein Polymer a) mit kationischen und/oder kationogenen Gruppen und einem Molekulargewicht M_{w} im Bereich von 10 000 bis 5 Millionen und
b) wenigstens ein Polymer b) ausgewählt aus
b1) Polymeren hergestellt in Gegenwart von Polymer a), die eine Verbindung der allgemeinen Formel I einpolymerisiert enthalten 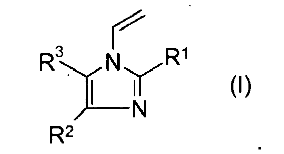 wobei R¹ bis R³ unabhängig voneinander Wasserstoff, C₁-C₄-Alkyl oder Phenyl bedeuten und
b2) Kationischen Polymeren, hergestellt in Gegenwart von Polymer a) und wenigstens einem Salz und einpolymerisiert enthaltend wenigstens ein kationisches Monomer und wenigstens eine Verbindung der allgemeinen Formel I.

2. Zusammensetzung nach Anspruch 1, wobei Polymer a) Diallyldimethylammoniumchlorid einpolymerisiert enthält.

3. Zusammensetzung nach einem der Ansprüche 1 oder 2, wobei Polymer a) zu wenigstens 50 Gew.-%, bevorzugt zu wenigstens 70 Gew.-% aus einpolymerisiertem Diallyldimethylammoniumchlorid besteht.

4. Zusammensetzung nach einem der Ansprüche 1 bis 3, wobei Polymer a) ein Molekulargewicht M_{w} im Bereich von 50 000 bis 500 000 g/mol, bevorzugt im Bereich von 100 000 bis 200 000 g/mol aufweist.

5. Zusammensetzung nach einem der Ansprüche 1 bis 4, wobei Polymer b1)
i) im Bereich von 10 bis 100 Gew.-% wenigstens einer Verbindung der allgemeinen Formel I und
ii) im Bereich von 0 bis 90 Gew.-% wenigstens einer Verbindung der allgemeinen Formel II 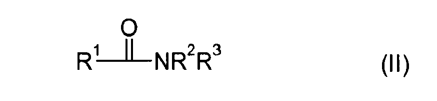
wobei R¹ für eine Gruppe der Formel CH₂=CR⁴- mit R⁴ = H oder C₁-C₄-Alkyl steht und R² und R³ unabhängig voneinander für H, Alkyl, Cycloalkyl, Heterocycloalkyl, Aryl oder Hetaryl stehen oder R² und R³ gemeinsam mit dem Stickstoffatom, an das sie gebunden sind, für einen fünf- bis achtgliedrigen Stickstoff-Heterocyclus stehen oder
R² für eine Gruppe der Formel CH₂=CR⁴- steht und R¹ und R³ unabhängig voneinander für H, Alkyl, Cycloalkyl, Heterocycloalkyl, Aryl oder Hetaryl stehen oder R¹ und R³ gemeinsam mit der Amidgruppe, an die sie gebunden sind für ein Lactam mit 5 bis 8 Ringatomen stehen,
einpolymerisiert enthält.

6. Zusammensetzung nach einem der Ansprüche 1 bis 5, wobei das Verhältnis der Gewichtsmengen der Polymere a) und b) in der Zusammensetzung im Bereich von 4:1 bis 1:4, bevorzugt im Bereich von 7:3 bis 3:7, besonders bevorzugt im Bereich von 3:2 bis 2:3 liegt.

7. Zusammensetzung nach einem der Ansprüche 1 bis 6, wobei Polymer b) zu wenigstens 10 Gew.-% aus einpolymerisiertem N-Vinylimidazol besteht.

8. Zusammensetzung nach einem der Ansprüche 1 bis 7, wobei Polymer b) zu wenigstens 40 Gew.-% aus einpolymerisiertem N-Vinylpyrrolidon besteht.

9. Zusammensetzung nach einem der Ansprüche 1 bis 8, wobei Polymer b1) ein Molekulargewicht M_{w} im Bereich von 1500 bis 500 000 g/mol aufweist.

10. Zusammensetzung nach einem der Ansprüche 1 bis 4, wobei das kationische Polymer b2) wenigstens eine quaternisierte Verbindung der allgemeinen Formel III einpolymerisiert enthält 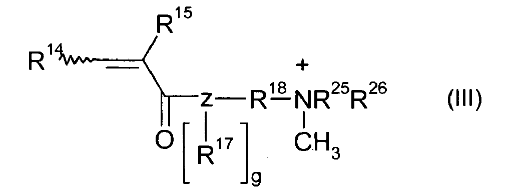 wobei
R¹⁴ und R¹⁵ unabhängig voneinander ausgewählt sind aus der Gruppe bestehend aus Wasserstoff, C₁-C₈ linear- oder verzweigtkettige Alkyl, Methoxy, Ethoxy, 2-Hydroxyethoxy, 2-Methoxyethoxy und 2-Ethoxyethyl,
R¹⁷ Wasserstoff oder Methyl ist,
R¹⁸ Alkylen oder Hydroxyalkylen mit 1 bis 24 C-Atomen, optional substituiert durch Alkyl, bevorzugt C₂H₄, C₃H₆, C₄H₈, CH₂-CH(OH)-CH₂ ist,
g 0 oder 1 ist,
Z Stickstoff wenn g = 1 oder Sauerstoff wenn g = 0 ist,
R²⁵ bzw. R²⁶ jeweils und unabhängig voneinander ausgewählt sind aus der Gruppe bestehend aus Wasserstoff, C₁-C₄₀ linear- oder verzweigtkettige Alkyl, Formyl, C₁-C₁₀ linear- oder verzweigtkettige Acyl, N,N-Dimethylaminoethyl, 2-Hydroxyethyl, 2-Methoxyethyl, 2-Ethoxyethyl, Hydroxypropyl, Methoxypropyl, Ethoxypropyl oder Benzyl.

11. Zusammensetzung nach Anspruch 10, wobei die quaternisierte Verbindung ausgewählt ist aus methyliertem N-[3-(dimethylamino)propyl]methacrylamid und methyliertem N,N-Dimethylaminoethylmethacrylat.

12. Zusammensetzung nach einem der Ansprüche 1 bis 11, wobei die Gesamtmenge von Polymer a) und Polymer b) in der Zusammensetzung im Bereich von 0,01 bis 20 Gew.-%, bezogen auf das Gewicht der Zusammensetzung, beträgt.

13. Zusammensetzung nach einem der Ansprüche 1 bis 12 enthaltend wenigstens ein anionisches Tensid.

14. Verwendung einer Zusammensetzung nach einem der Ansprüche 1 bis 13 zur Pflege der Haare.

15. Haarpflegemittel enthaltend eine Zusammensetzung nach einem der Ansprüche 1 bis 13.

16. Hautkosmetisches Mittel enthaltend eine Zusammensetzung nach einem der Ansprüche 1 bis 13.

17. Haarpflegemittel nach Anspruch 15 in Form eines Shampoos.

18. Verfahren zur Herstellung einer Zusammensetzung gemäß einem der Ansprüche 1 bis 13, **dadurch gekennzeichnet, dass** Polymer b1) durch Lösungspolymerisation in Gegenwart von Polymer a) hergestellt wird.

## Claims

1. A composition comprising
a) at least one polymer a) with cationic and/or cationogenic groups and a molecular weight M_{w} in the range from 10 000 to 5 million and
b) at least one polymer b) selected from
b1) polymers prepared in the presence of polymer a) which comprise a compound of the general formula I in copolymerized form 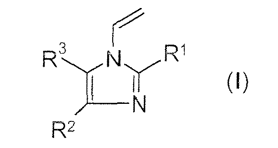 where R¹ to R³, independently of one another, are hydrogen, C₁-C₄-alkyl or phenyl, and
b2) cationic polymers prepared in the presence of polymer a) and at least one salt and comprising, in copolymerized form, at least one cationic monomer and at least one compound of the general formula I.

2. The composition according to claim 1, where polymer a) comprises diallyldimethylammonium chloride in copolymerized form.

3. The composition according to one of claims 1 or 2, where polymer a) consists of at least 50% by weight, preferably of at least 70% by weight, of copolymerized diallyldimethylammonium chloride.

4. The composition according to one of claims 1 to 3, where polymer a) has a molecular weight M_{w} in the range from 50 000 to 500 000 g/mol, preferably in the range from 100 000 to 200 000 g/mol.

5. The composition according to one of claims 1 to 4, where polymer b1) comprises, in copolymerized form,
i) in the range from 10 to 100% by weight, at least one compound of the general formula I and
ii) in the range from 0 to 90% by weight, at least one compound of the general formula II 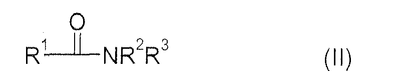 where R¹ is a group of the formula CH₂=CR⁴- where R⁴ = H or C₁-C₄-alkyl, and R² and R³, independently of one another, are H, alkyl, cycloalkyl, heterocycloalkyl, aryl or hetaryl, or R² and R³, together with the nitrogen atom to which they are bonded, are a five- to eight-membered nitrogen heterocycle or R² is a group of the formula CH₂=CR⁴-, and R¹ and R³, independently of one another, are H, alkyl, cycloalkyl, heterocycloalkyl, aryl or heteraryl, or R¹ and R³, together with the amide group to which they are bonded, are a lactam having 5 to 8 ring atoms.

6. The composition according to one of claims 1 to 5, where the ratio of the weight amounts of the polymers a) and b) in the composition is in the range from 4:1 to 1:4, preferably in the range from 7:3 to 3:7, particularly preferably in the range from 3:2 to 2:3.

7. The composition according to one of claims 1 to 6, where polymer b) consists of at least 10% by weight of copolymerized N-vinylimidazole.

8. The composition according to one of claims 1 to 7, where polymer b) consists of at least 40% by weight of copolymerized N-vinylpyrrolidone.

9. The composition according to one of claims 1 to 8, where polymer b1) has a molecular weight M_{w} in the range from 1500 to 500 000 g/mol.

10. The composition according to one of claims 1 to 4, where the cationic polymer b2) comprises at least one quaternized compound of the general formula III in copolymerized form 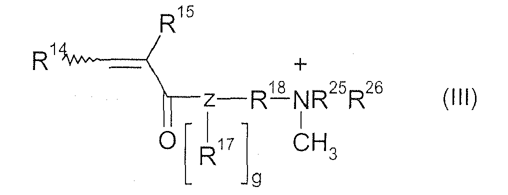 where
R¹⁴ and R¹⁵, independently of one another, are selected from the group consisting of hydrogen, C₁-C₈ linear- or branched-chain alkyl, methoxy, ethoxy, 2-hydroxyethoxy, 2-methoxyethoxy and 2-ethoxyethyl,
R¹⁷ is hydrogen or methyl,
R¹⁸ is alkylene or hydroxyalkylene having 1 to 24 carbon atoms, optionally substituted by alkyl, preferably C₂H₄, C₃H₆, C₄H₈, CH₂-CH(OH)-CH₂,
g is 0 or 1,
Z is nitrogen when g = 1 or oxygen when g = 0,
R²⁵ and R²⁶, in each case and independently of one another, are selected from the group consisting of hydrogen, C₁-C₄₀ linear- or branched-chain alkyl, formyl, C₁-C₁₀ linear- or branched-chain acyl, N,N-dimethylaminoethyl, 2-hydroxyethyl, 2-methoxyethyl, 2-ethoxyethyl, hydroxypropyl, methoxypropyl, ethoxypropyl or benzyl.

11. The composition according to claim 10, where the quaternized compound is selected from methylated N-[3-(dimethylamino)propyl]methacrylamide and methylated N,N-dimethylaminoethyl methacrylate.

12. The composition according to one of claims 1 to 11, where the total amount of polymer a) and polymer b) in the composition is in the range from 0.01 to 20% by weight, based on the weight of the composition.

13. The composition according to one of claims 1 to 12, comprising at least one anionic surfactant.

14. The use of a composition according to one of claims 1 to 13 for haircare.

15. A haircare composition comprising a composition according to one of claims 1 to 13.

16. A skin cosmetic composition comprising a composition according to one of claims 1 to 13.

17. The haircare composition according to claim 15 in the form of a shampoo.

18. A method of producing a composition according to one of claims 1 to 13, wherein polymer b1) is produced by solution polymerization in the presence of polymer a).

## Revendications

1. Composition contenant
a) au moins un polymère a) comportant des groupes cationiques et/ou cationogènes et ayant une masse moléculaire M_{w} dans la plage de 10 000 à 5 millions et
b) au moins un polymère b) choisi parmi
b1) des polymères préparés en présence du polymère a), qui contiennent incorporé par polymérisation un composé de formule générale I 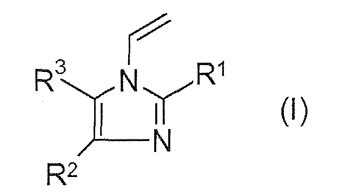 dans laquelle R¹ à R³ représentent chacun indépendamment un atome d'hydrogène, un groupe alkyle en C₁-C₄ ou phényle et
b2) des polymères cationiques, préparés en présence du polymère a) et d'au moins un sel et qui contiennent incorporé par polymérisation au moins un monomère cationique et au moins un composé de formule générale I.

2. Composition selon la revendication 1, dans laquelle le polymère a) contient incorporé par polymérisation du chlorure de diallyldiméthylammonium.

3. Composition selon la revendication 1 ou 2, dans laquelle le polymère a) consiste à raison d'au moins 50 % en poids, de préférence d'au moins 70 % en poids en chlorure de diallyldiméthylammonium incorporé par polymérisation.

4. Composition selon l'une quelconque des revendications 1 à 3, dans laquelle le polymère a) présente une masse moléculaire M_{w} dans la plage de 50 000 à 500 000 g/mole, de préférence dans la plage de 100 000 à 200 000 g/mole.

5. Composition selon l'une quelconque des revendications 1 à 4, dans laquelle le polymère b1) contient incorporés par polymérisation
i) dans la plage de 10 à 100 % en poids d' au moins un composé de formule générale I et
ii) dans la plage de 0 à 90 % en poids d'au moins un composé de formule générale II 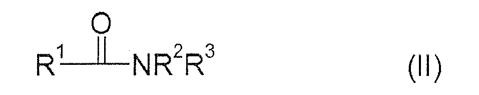
dans laquelle R¹ représente un groupe de formule CH₂=CR⁴- où R⁴ = H ou un groupe alkyle en C₁-C₄ et R² et R³ représentent, indépendamment l'un de l'autre, H, un groupe alkyle, cycloalkyle, hétérocycloalkyle, aryle ou hétéroaryle, ou R² et R³ représentent, conjointement avec l'atome d'azote auquel ils sont liés, un hétérocycle azoté à cinq à huit chaînons ou
R² représente un groupe de formule CH₂=CR⁴- et R¹ et R³ représentent, indépendamment l'un de l'autre, H, un groupe alkyle, cycloalkyle, hétérocycloalkyle, aryle ou hétéroaryle, ou R¹ et R³ représentent, conjointement avec le groupe amido auquel ils sont liés, un lactame ayant de 5 à 8 atomes formant le cycle.

6. Composition selon l'une quelconque des revendications 1 à 5, dans laquelle le rapport des quantités pondérales des polymères a) et b) dans la composition se situe dans la plage de 4:1 à 1:4, de préférence dans la plage de 7:3 à 3:7, de façon particulièrement préférée dans la plage de 3:2 à 2:3.

7. Composition selon l'une quelconque des revendications 1 à 6, dans laquelle le polymère b) consiste à raison d'au moins 10 % en poids en N-vinylimidazole incorporé par polymérisation.

8. Composition selon l'une quelconque des revendications 1 à 7, dans laquelle le polymère b) consiste à raison d'au moins 40 % en poids en N-vinylpyrrolidone incorporée par polymérisation.

9. Composition selon l'une quelconque des revendications 1 à 8, dans laquelle le polymère b1) présente une masse moléculaire M_{w} dans la plage de 1 500 à 500 000 g/mole.

10. Composition selon l'une quelconque des revendications 1 à 4, dans laquelle le polymère cationique b2) contient incorporé par polymérisation au moins un composé rendu quaternaire de formule générale III 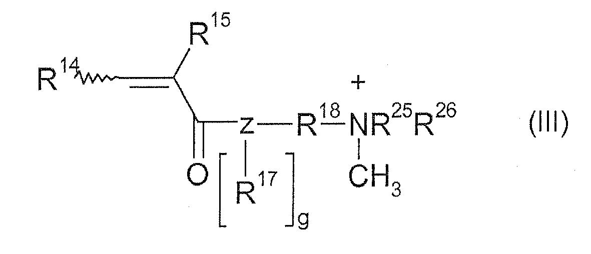 où
R¹⁴ et R¹⁵ sont choisis, indépendamment l'un de l'autre, dans l'ensemble constitué par un atome d'hydrogène, un groupe alkyle en C₁-C₈ à chaîne droite ou ramifiée, méthoxy, éthoxy, 2-hydroxyéthoxy, 2-méthoxyéthoxy ou 2-éthoxyéthyle,
R¹⁷ est un atome d'hydrogène ou le groupe méthyle,
R¹⁸ est un groupe alkylène ou hydroxyalkylène ayant de 1 à 24 atomes de carbone, éventuellement substitué par alkyle, de préférence C₂H₄, C₃H₆, C₄H₈, CH₂-CH(OH)-CH₂,
g est 0 ou 1,
Z est un atome d'azote lorsque g = 1 ou un atome d'oxygène lorsque g = 0,
R²⁵ et R²⁶ sont choisis chacun et indépendamment l'un de l'autre dans l'ensemble constitué par un atome d' hydrogène, un groupe alkyle en C₁-C₄₀ à chaîne droite ou ramifiée, formyle, acyle en C₁-C₁₀ à chaîne droite ou ramifiée, N,N-diméthylaminoéthyle, 2-hydroxyéthyle, 2-méthoxyéthyle, 2-éthoxyéthyle, hydroxypropyle, méthoxypropyle, éthoxypropyle ou benzyle.

11. Composition selon la revendication 10, dans laquelle le composé rendu quaternaire est choisi parmi le N-[3-(diméthylamino)propyl]méthacrylamide méthylé et le méthacrylate de N,N-diméthylaminoéthyle méthylé.

12. Composition selon l'une quelconque des revendications 1 à 11, dans laquelle la quantité totale du polymère a) et du polymère b) dans la composition se situe dans la plage allant de 0,01 à 20 % en poids, par rapport au poids de la composition.

13. Composition selon l'une quelconque des revendications 1 à 12, contenant au moins un tensioactif anionique.

14. Utilisation d'une composition selon l'une quelconque des revendications 1 à 13, pour le soin des cheveux.

15. Produit de soin capillaire, contenant une composition selon l'une quelconque des revendications 1 à 13.

16. Produit cosmétique pour la peau, contenant une composition selon l'une quelconque des revendications 1 à 13.

17. Produit de soin capillaire selon la revendication 15, sous forme d'un shampooing.

18. Procédé pour la préparation d'une composition selon l'une quelconque des revendications 1 à 13, **caractérisé en ce que** le polymère b1) est préparé par polymérisation en solution en présence du polymère a).
